# FASCICULE DE BREVET EUROPEEN

(11) **EP 1 025 121 B1**
(45) Date de publication et mention de la délivrance du brevet: **24.08.2011**
(21) Numéro de dépôt: 98951547.3
(22) Date de dépôt: 23.10.1998
(51) Int. Cl.: C07K 14/47, C12N 15/12, C12N 5/10, C12N 15/86, C07K 16/18

(54) **PEPTIDES CAPABLES D'INHIBER L'INTERACTION ENTRE LES PRESENILINES ET LE PEPTIDE BETA-AMYLOIDE OU SON PRECURSEUR**
PEPTIDE DIE DIE WECHSELWIRKUNG ZWISCHEN PRESENILINEN UND BETA-AMYLOIDPEPTID ODER SEINEM VORLÄUFER HEMMEN
PEPTIDES CAPABLE OF INHIBITING THE INTERACTION BETWEEN PRESENILINS AND THE BETA-AMYLOID PEPTIDE OR ITS PRECURSOR

(30) Priorité: 24.10.1997 FR 9713384; 07.08.1998 US 95671 P
(43) Date de publication de la demande: 09.08.2000
(73) Titulaire: Aventis Pharma S.A., 92160 Antony (FR)
(72) Inventeur: CZECH, Christian, F-75007 Paris (FR); MERCKEN, Luc, F-94100 Saint Maur (FR); PRADIER, Laurent, F-91370 Verrières (FR); REBOUL-BECQUART, Soline, F-92120 Montrouge (FR)
(74) Mandataire: Bouvet, Philippe
(86) Numéro de dépôt international: PCT/FR1998/002278
(87) Numéro de publication internationale: WO 1999/021886

(56) Documents cités:
- WO-A-92/13069
- WO-A-96/34099
- WO-A-97/27296
- XIA W ET AL: "Interaction between amyloid precursor protein and presenilins in mammalian cells: Implications for the pathogenesis of Alzheimer disease" PROCEEDINGS OF THE NATIONAL ACADEMY OF SCIENCES OF USA., vol. 94, juillet 1997, pages 8208-8213, XP002067171 WASHINGTON US cité dans la demande
- KIM S S ET AL: "Lack of interactions between amyloid precursor protein and hydrophilic domains of presenilin 1 and 2 using the yeast two hybrid system" JOURNAL OF MOLECULAR NEUROSCIENCE, vol. 9, no. 1, août 1997, pages 49-54, XP002072945 TOTOWA US
- WEIDEMANN A ET AL.: "Formation of stable complexes between two Alzheimer's disease gene products: Presenilin-2 and beta amyloid precursor protein" NATURE MEDICINE., vol. 3, no. 3, mars 1997, pages 328-332, XP002067172 NEW YORK US cité dans la demande
- WARAGAI M ET AL.: "Presenilin 1 binds to amyloid precursor protein directly" BIOCHEMICAL AND BIOPHYSICAL RESEARCH COMMUNICATIONS., vol. 239, 20 octobre 1997, pages 480-482, XP002094076 ORLANDO, FL US
- WEIDEMANN A ET AL.: "Modulation of APP metabolism by presenilin-2 derivatives" SOCIETY FOR NEOROSCIENCE ABSTRACTS, vol. 23, no. 1-2, 25 octobre 1997, page 1118 XP002094077 US
- HAAS C ET AL.: "A technical KO of amyloid-beta peptide" NATURE., vol. 391, 22 janvier 1998, pages 339-340, XP002094078 LONDON GB
- THINAKARAN G ET AL: "Stable association of presenilin derivatives and absence of presenilin interactions with APP" NEUROBIOLOGY OF DISEASE, vol. 4, no. 6, 1998, pages 438-453, XP002072947 NEW YORK US

## Description

La présente invention concerne l'élaboration de tests in vitro pour la mise en évidence de molécules et en particulier de petites molécules capables d'inhiber l'interaction entre l'APP et l'extremité N-Terminale des Presenilines.

Le peptide amyloïde Aβ, de 37 à 42 acides aminés, est le principal composant protéique des plaques séniles caractéristiques de la maladie d'Alzheimer. Ce peptide est produit par clivage de son précurseur, la protéine précurseur de peptide amyloïde (APP). Des mutations dans le géne de l'APP sont responsables de certaines formes familiales précoces de la maladie d'Alzheimer. Cependant la majorité de ces formes sont liées à la présence de mutations sur deux gènes, présénilines PS1 (initialement nommé S182) et PS2 (initialement STM2), récemment identifiés par clonage positionnel (Hardy, 1997). Ces formes sont dominantes et ces anomalies correspondent toutes à des mutations faux-sens à l'exception d'une entrainant la délétion d'un exon. Les présénilines sont des protéines hydrophobes membranaires d'environ 45-50 kDa de masse moléculaire et qui présentent 67% d'identité entre elles. Elles sont homologues à deux protéines de C. elegans, SPE4 et Sel-12, qui sont impliquées de façon indirecte respectivement dans le transport intracellulaire et dans la signalisation des récepteurs Notch. Cependant, la fonction physiologique des présénilines est encore inconnue. L'implication dans la maladie d'Alzheimer, de deux protéines si voisines, laisse à penser que les présénilines contribuent à une voie physiologique essentielle dans l'étiologie de cette pathologie.

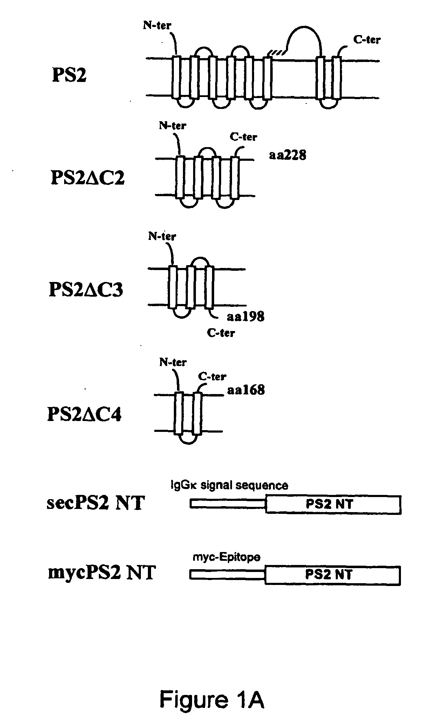

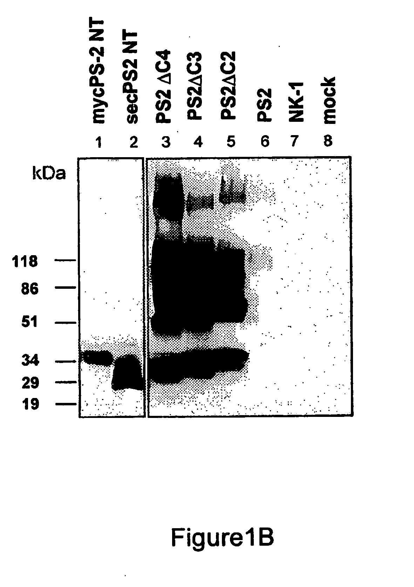

La protéine PS1 comprend 467 acides aminé et PS2 448. Toutes deux présentent la structure d'une protéine membranaire avec de 6 à 8 domaines transmembranaires potentiels. Chacune des présénilines est sujette in vivo à un clivage protéolytique précis résultant en deux fragments généralement dénommés fragments N(amino)- et C(carboxy)-terminaux (Thinakaran et col., 1996). Ce clivage a été cartographié entre les résidus 291 et 299 de PS1 (Podlisny et col., 1997) et dans une région homologue de PS2. On entend donc en général par fragment N-terminal (N-ter), le fragment de la position 1 à approximativement 291 de PS1 et par fragment C-terminal, le complément. Bien que la topologie exacte des présénilines dans les membranes lipidiques ne soit pas clairement établie, il est proposé que leurs extrémités N- et C-terminales[RC1], ainsi que la grande boucle hydrophile, soient présentes dans le compartiment cytosolique (Doan et col, 1996, voir schéma figure 1).

Il a maintenant été démontré que les formes mutées des présénilines induisent l'augmentation de la production du peptide amyloide long Aβ 1-42 par rapport à celle de Aβ 1-40 autant chez les patients porteurs (Scheuner et coll., 1996), qu'en cellules transfectées (Borchelt et coll., 1996) ou en souris transgéniques (Duff et coll: 1996). Le peptide amyloide Aβ, qui forme les plaques séniles, lésions caractéristiques de la pathologie, et ses différentes formes sont dérivés du catabolisme de la protéine précurseur de l'amyloïde, APP. En particulier, deux formes essentielles du peptide amyloide ont été décrites, une de quarante résidus, Aβ40, et l'autre possédant deux résidus supplémentaires en son carboxy terminal, Aβ42. In vitro, le peptide Aβ présente de fortes propriétés d'agrégation qui sont accrues pour la forme Aβ42 et ce dernier semble effectivement former les premiers agrégats détectables dans la pathologie. Par ailleurs, la forme Aβ42 est spécifiquement produite après trauma crânien chez l'homme, lequel constitue un des facteurs de risques environnementaux les mieux établis de la maladie d'Alzheimer. De plus, les formes génétiques précoces de la maladie liées à des mutations autant sur l'APP (il y en a six) que maintenant sur les présénilines 1 et 2, concourent toutes à une augmentation du ratio Aβ42/Aβ40. L'ensemble de ces facteurs semble désigner le Aβ42 comme l'agent clé de la pathologie autant dans les formes génétiques que sporadiques de la maladie et l'élucidation de son mécanisme de formation est devenu une question fondamentale.

A cet égard, la formation de complexe dans la même enveloppe cellulaire entre le précurseur du peptide β-amyloïde et PS1 ou PS2 a été rapportée (Weidemann et al. 1997, Xia et al., 1997) cependant on ne connaît pas la nature précise des événements responsables de la production du peptide β-amyloide et aucun lien n'a encore pu être établi entre le rôle possible de ces complexes et la production du peptide β-amyloïde Aβ42. Il est néanmoins important de remarquer que le peptide Aβ42, mais non pas le Aβ40, semble être localisé dans le reticulum endoplasmique dans les cellules neuronales (Hartmann et al., 1997).

La présente invention résulte de l'identification et de la caractérisation par la demanderesse de régions particulières de la préséniline 1 (PS1) et de la préséniline 2 (PS2), ainsi que de régions particulières du précurseur du peptide β-amyloïde (APP) impliquées dans la formation de complexes APP/PS1 et APP/PS2.

La présente invention découle en particulier de la mise en évidence de la capacité de la région N-terminale hydrophile (acides aminés 1-87) de la PS2 à reconnaître différents domaines de APP. Elle découle en outre de la mise en évidence de propriétés similaires pour la région N-terminale de PS1 (fragment 1-213). Elle résulte également de la démonstration de la capacité des polypeptides dérivés des régions des présénilines définies ci-avant à inhiber la formation des complexes entre l'APP et les présénilines. Les présénilines de la présente demande correspondent essentiellement à la préséniline 1 (PS1) et/ou à la préséniline 2 (PS2).

La présente invention résulte en outre de la mise en évidence de la localisation cellulaire particulière, non attendue, des régions en interaction par rapport à la membrane lipidique. Elle découle plus particulièrement du fait que ces interactions peuvent prendre place non seulement au niveau membranaire mais également au niveau de la lumière du reticulum endoplasmique et dans le compartiment extracellulaire. Ceci est inattendu dans la mesure où la région N-terminale des PS (impliquée dans l'interaction) est généralement considérée comme étant localisée dans le cytoplasme dans des conditions standard.

La caractérisation des domaines d'interaction de l'APP et des présénilines et la mise en évidence des différentes localisations cellulaires de ces interactions permettent d'envisager la préparation de nouveau polypeptides utilisables pharmaceutiquement. Un premier objet de l'invention concerne un procédé de mise en évidence ou ou d'indement de composés destinés au traitement de la maladie d'Alzheimer comprenant au moins une étape de détection de l'inhibition de l'interaction entre l'APP et un peptide comprenant la séquence d'acides aminés 1 à 213 de PS1 ou la séquence d'acides aminés 1 à 87 de PS2.

Au sens de capable d'inhiber l'interaction, on entend que la présence des polypeptides de l'invention et/ou des ligands et/ou molécules mis en évidence à l'aide du procédé de l'invention, suffisent à inhiber au moins partiellement la dite interaction entre une préséniline et/ou son extrémité N-Terminale et le précurseur du peptide β-amyloïde et/ou le peptide β-amyloïde et de préférence le peptide Aβ₁. ₁₂.

Il est démontré dans les exemples de la présente demande que l'inhibition de cette interaction avec un des polypeptides de l'invention, conduit à la diminution de la production du peptide amyloide intracellulaire Aβ₁₋₁₂. Cette conséquence fonctionnelle est donc envisagée pour tout polypeptide de l'invention et/ou ligands et/ou molécules mis en évidence à l'aide du procédé de l'invention. Inhiber cette interaction et donc inhiber la production de Aβ₁₋₁₂, représente par conséquent une cible thérapeutique de choix dans les maladies impliquant cette forme du peptide amyloide.

Selon un mode particulier, les polypeptides mis en évidence par l'invention comportent au moins une partie de la préséniline 2 (PS2) permettant l'interaction avec le précurseur du peptide β-amyloïde et/ou le peptide β-amyloïde. De manière préférée, les polypeptides sont caractérisés en ce que la partie de PS2 correspond au fragment N-terminal hydrophile de PS2. Plus préférentiellement les polypeptides comprennent tout ou partie de la séquence correspondant à la séquence SEQ ID N°1 ou d'une séquence dérivée de celle-ci.

Selon un autre mode de réalisation, les polypeptides mis en évidence par l'invention comportent au moins une partie de PS1 permettant l'interaction avec le précurseur du peptide β-amyloïde et/ou le peptide β-amyloïde. De manière préférée les polypeptides selon l'invention comprennent tout ou partie de la séquence correspondant à la séquence SEQ ID N°2 ou d'une séquence dérivée de celle-ci.

Selon un autre mode de réalisation les polypeptides comprennent au moins les régions d'homologie communes correspondant aux séquences SEQ IDN° 1 et SEQ ID N°2.

Selon un autre mode de réalisation les polypeptides mis en evidence par l'invention comportent au moins une partie du précurseur du peptide β-amyloïde (APP). De manière préférée, les polypeptides comportent une partie de l'APP hormis la région correspondant au peptide β-amyloïde. Encore plus préférentiellement, les polypeptides sont caractérisés en ce que la partie du précurseur du peptide β-amyloïde comprend tout ou partie du fragment 1-596. Plus préférentiellement, les polypeptides comportent tout ou partie d'une séquence choisie parmi-la séquence correspondant au fragment 1-596 de la séquence SEQ ID N°3, ou une séquence dérivée.

Au sens de la présente invention, le terme séquence polypeptidique dérivée désigne toute séquence polypeptidique différant des séquences polypeptidiques correspondant aux séquences présentées en SEQ ID N°1 ou SEQ ID N°2, ou les fragments désignés de la SEQ ID N°3, obtenue par une ou plusieurs modifications de nature génétique et/ou chimique, et possédant la capacité d'inhiber au moins en partie l'interaction entre la préséniline 1 ou la préséniline 2 et le précurseur du peptide β-amyloïde et/ou le peptide β-amyloïde. Par modification de nature génétique et/ou chimique, on doit entendre toute mutation, substitution, délétion, addition et/ou modification d'un ou plusieurs résidus. De tels dérivés peuvent être générés dans des buts différents, tels que notamment celui d'augmenter l'affinité du peptide pour son site d'interaction, celui d'améliorer son niveau de production, celui d'augmenter sa résistance aux protéases, celui d'augmenter son efficacité thérapeutique ou de réduire ses effets secondaires, ou celui de lui conférer de nouvelles propriétés pharmacocinétiques et/ou biologiques.

L'invention concerne également la mise en évidence ou l'isolement des composés non peptidiques ou non exclusivement peptidiques utilisables pharmaceutiquement. Il est en effet possible, à partir des motifs polypeptidiques décrits dans la présente demande, de réaliser des molécules qui inhibent au moins partiellement l'interaction entre la préséniline 1 ou la préséniline 2 et le précurseur du peptide β-amyloïde et/ou le peptide β-amyloïde, et qui soient non exclusivement peptidiques et compatibles avec une utilisation pharmaceutique. L'utilisation de polypeptides tels que décrits ci-avant pour la préparation de molécules non peptidiques, ou non exclusivement peptidiques, actives pharmacologiquement, par détermination des éléments structuraux de ces polypeptides qui sont importants pour leur activité et reproduction de ces éléments par des structures non peptidiques ou non exclusivement peptidiques peut-être envisagée.

Les polypeptides mis en évidence par l'invention doivent comprendre des séquences permettant une localisation cellulaire précise afin d'inhiber l'interaction entre les présénilines et le précurseur du peptide β-amyloïde et/ou le peptide β-amyloïde Préférentiellement ce sont les polypeptides dérivés de SEQ ID 1 et SEQ ID 2 qui comprenant des séquences de localisation cellulaire exogène et de manière encore plus préférée un polypeptide comprenant l'extrémité N-Terminale de PSI ou de PS2. Parmi ces séquences on peut citer les séquences de peptide signal telles que la séquence du peptide signal de IgkB, le peptide signal de l'APP, les peptides signal des sous-unités des récepteurs nicotiniques de l'acétylcholine musculaires et centraux etc...

Parmi les polypeptides particulièrement intéressants on peut cité un polypeptide comprenant les 87 premiers résidus de l'extrémité N-Terminale de PS2 et le peptide signal de IgkB.

L'objet de la présente invention concerne en particulier un procédé d'identification de composés capables de moduler ou d'inhiber au moins en partie l'interaction entre la préséniline 1 ou la préséniline 2 et le précurseur du peptide β-amyloïde et/ou le peptide β-amyloïde. En particulier, le procédé est utilisable comme test de criblage de molécules pour identifier de tels composés inhibiteurs.

Ce test repose en particulier sur la détection de l'inhibition de l'interaction d'une manière générale entre les présénilines (1 ou 2) et l'APP ou le peptide Aβ et d'une manière particulière entre le peptide Aβ₁₋₁₂ et l'extrémité N-Terminale de PS2 En effet, à l'aide de protéines marqueur fixées aux présénilines ou des fragments de celles-ci et de systèmes de révélation appropriés et notamment par immunoprécipitation, utilisation de chromophores ou fluophores, il est tout à fait possible de détecter une inhibition dans l'interaction des protéines ou fragments de celles-ci, précédemment cités . Un tel procédé comprend donc au moins une étape de marquage des présénilines et/ou de l'APP ou des fragments de ceux-ci et une étape de détection de l'inhibition de l'interaction soit entre le peptide Aβ₁₋₁₂ et l'extrémité N-terminale des présénilines et préférentiellement PS2, soit entre les protéines complètes APP et présénilines.

Selon un premier mode de réalisation du procidé, la mise en évidence et/ou identification de tels composés est réalisée selon les étapes suivantes :
- Le peptide Aβ₁₋₁₂ est absorbé au préalable sur une membrane de nitrocellulose par incubation.
- un extrait bactérien contenant tout ou partie d'une préséniline (PS1 ou PS2) et avantageusement l'extrémité N-Terminale, est ensuite ajouté pour incubation avec la molécule ou un mélange contenant différentes molécules à tester
- Après lavages, l'interaction de la préséniline avec le peptide Aβ₁₋₄₂ sur le filtre de nitrocellulose est mise en évidence à l'aide de protéines marqueur des présénilines. Les molécules recherchées inhibent l'interaction et diminuent donc l'intensité du signal des protéines marqueur.

Les protéines marqueur utilisées sont avantageusement a) la protéine fixatrice du S-tag, couplée à la phosphatse alkaline ou à un chromophore fluorescent, ou b) un anticorps anti-PSNT c'est-à-dire dirigé contre l'extrémité N-Terminal d'une préséniline.

Selon une autre mode de réalisation du procédé, la recherche de nouveaux composés est effectuée de la manière suivante :
- le peptide Aβ42 incubé au préalable sur une plaque contenant des puits (format 96 puits ou supérieur)
- l'extrémité N-terminale d'une préséniline recombinante purifiée est ensuite ajoutée avec la molécule ou un mélange contenant différentes molécules à tester, pour incubation
- Après lavages, l'interaction de la préséniline avec le peptide Aβ₁₋₄₂ dans la plaque est mise en évidence à l'aide de protéines marqueur des présénilines. La perte de l'interaction entre les présénilines et le précurseur du peptide β-amyloïde et/ou le peptide β-amyloïde est détectée par spectrophotomètrie.
Dans le cas précis de l'utilisation de la protéine fixatrice du S-tag couplée à la phosphatase alkaline comme protéine marqueur, après révélation avec un substrat colorimétrique, le signal est détecté à 450 nm .

Selon un mode de réalisation avantageux et préféré du procédé, la mise en évidence et/ou l'isolement de composés capables de moduler ou d'inhiber au moins en partie l'interaction d'une manière générale entre la préséniline 1 ou la préséniline 2 et le précurseur du peptide β-amyloïde et/ou le peptide β-amyloïde et d'une manière particulière entre le peptide Aβ₁₋₄₂ et l'extrémité N-Terminale de PS2, est réalisé selon les étapes suivantes:
- on met en contact une molécule ou un mélange contenant différentes molécules avec le peptide Aβ₁₋₄₂ synthétisé avec une biotine et un bras de 3 βAlanines (ou de 3 lysines) à son extrémité N-terminale (en amont de la position 1)
- on incube le mélange réactionnel précédent avec l'extrémité N-terminale d'une préséniline purifiée marquée à l'aide d'un premier fluorophore. Avantageusement, le fluophore est le cryptate d'Europium
- on ajoute la streptavidine (qui se fixera sur la biotine du peptide biot-Aβ₁₋₄₂) couplée à un second fluophore, capable d'être excité à la longueur d'onde d'émission du premier fluophore afin qu'il bénéficie d'un transfert de fluorescence si les deux fluorophores se trouvent à forte proximité.
- la mise en évidence des nouveaux composés inhibant l'interaction est détectée par fluoromètrie à la longueur d'émission du premier fluophore et/ou en mesurant la diminution du signal à la longueur d'onde d'émission du second fluophore

Selon un mode particulier, ce second fluophore est la XL665 qui est l'allophycocyanine crosslinkée chimiquement pour augmenter sa fluorescence à 665 nm (CisBiointernational). La perte de l'interaction est donc est détectée par fluorométrie à la longueur d'onde d'émission du premier fluophore et par la diminution du signal de la XL665 dont la longueur d'onde d' émission est à 665 nm.

Ce procédé est tout à fait avantageux car il permet de mettre en évidence directement l'interaction entre les présénilines et l'APP et/ou le peptide Aβ en phase liquide et homogène et par conséquent mettre en évidence les molécules inhibitrices de ladite interaction. En effet, ce procédé repose sur le transfert de fluorescence entre deux fluophores si ces deux chromophores sont en proximité physique (donc en cas d'interaction entre Aβ₁₋₄₂ et la protéine recombinante). Selon une variante préférée du procédé, le premier fluophore est le cryptate d'Europium, porté par la protéine recombinante marquée (excité à 337 nm) réagissant avec la streptavidine-XL665 fixée sur le peptide biot-Aβ, et en particulier le peptide biot-A_{β1-42}. Avantageusement, la protéine marquée est constituée par l'extrémité N-Terminale de l'une ou l'autre des présénilines (PSNT-K). La perte de la fluorescence à 665 nm et l'augmentation de la fluorescence à 620 nm caractéristique du cryptate d'Europium indique une inhibition de l'interaction entre les présénilines ou leurs extrémités N-Terminales et l'APP et/ou le peptide Aβ par les molécules recherchées.

Selon une variante de ce procédé, la molécule ou le mélange contenant les différentes molécules peuvent être mis en contact d'abord avec l'extrémité N-terminale d'une préséniline purifiée marquée à l'aide du cryptate d'Europium (PSNT-K) puis avec le peptide Aβ1-40 ou Aβ1-42 portant une biotine et un bras de 3 βAlanines (ou de 3 lysines) à leur extrémité N-terminale. Le mise en évidence de nouvelle molécules capables de moduler ou d'inhiber au moins en partie l'interaction entre la préséniline 1 ou la préséniline 2 et le précurseur du peptide β-amyloïde et/ou le peptide β-amyloïde sera faite également, après adjonction de la streptavidine marquée à la XL665, par spectrofluorométrie selon le procédé précédent et notamment par lecture de la fluorescence à 665 nm.

Selon un dernier mode de réalisation du procédé de mise en évidence de composés inhibant l'interaction d'une manière générale entre les présénilines (1 ou 2) et l'APP ou le peptide Aβ et d'une manière particulière entre la peptide Aβ₁₋₄₂ et l'extrémité N-Terminale de PS2 comporte les étape suivantes :
- on met en contact un mélange a) de lysats cellulaires contenant tout ou partie d'une préséniline (PS1 ou PS2) et avantageusement l'extrémité N-Terminale, b) de lysats cellulaires contenant l'APP, lysats obtenus à partir de cellules infectées par des virus et en particulier par des baculovirus et c) la molécule ou un mélange contenant différentes molécules à tester
- on co-immunoprécipite à l'aide d'anticorps appropriés et bien connus de l'homme du métier, les protéines solubilisées et correspondant aux présénilines ou à l'APP ou le peptide A β
- la perte de la co-immunoprécipitation des présénilines et de l'APP est révélée par western blot avec des anticorps marqueurs indiquant que les molécules testées ont la propriété inhibitrice recherchée

Dans un mode particulier, les procédés de l'invention décrits précédemment sont adaptés à la mise en évidence et/ou l'isolement de ligands, agonistes ou antagonistes de l'interaction entre les présenilines et le précurseur du peptide β-amyloïde et/ou le peptide β-amyloïde.

La présente invention concerne également l'utilisation des polypeptides définis précédemment pour la mise en évidence de ligands des polypeptides mais surtout de ligands des présénilines, du précurseur du peptide β-amyloïde et/ou du peptide β-amyloïde, et préférentiellement du peptide Aβ₁₋₄₂ et/ou de l'extrémité N-Terminale de PS2, ainsi que de composés capables d'inhiber au moins en partie l'interaction entre une préséniline et le précurseur du peptide β-amyloïde et/ou le peptide β-amyloïde

Un autre objet de l'invention concerne l'utilisation d'un ligand ou d'un modulateur identifié et/ou obtenu selon les procédés décrits ci-avant comme médicament. De tels ligands ou modulateurs de par leur capacité à interférer au niveau de l'interaction entre les présenilines et le précurseur du peptide β-amyloïde et/ou le peptide β-amyloïde peuvent donc moduler la production du peptide amyloide Aβ₁₋₄₂ et permettre de traiter certaines affections neurologiques et notamment la maladie d'Alzheimer.

Un autre objet de l'invention concerne la mise au point d'un test d'interaction entre une préséniline et le précurseur du peptide β-amyloïde et/ou le peptide β-amyloïde, et préférentiellement entre le peptide Aβ₁₋₄₂ et l'extrémité N-Terminale de PS2, caractérisé en ce qu'il comprend au moins une étape de transfert de fluorescence entre deux fluophores fixés aux molécules précédentes et une étape de révélation de l'interaction mesurée par spectrofluorométrie. Comme mentionné précédemment ce test est également utilisé pour la mise en évidence de molécules inhibitrices de la dite interaction, selon le procédé de détection de l'inhibition de l'interaction, décrit dans la présente demande.

L'invention a encore pour objet toute composition pharmaceutique comprenant comme principe actif au moins un polypeptide mis en évidence pour l'invention.

Les compositions pharmaceutiques selon l'invention peuvent être utilisées pour inhiber au moins en partie l'interaction entre une préséniline et le précurseur du peptide β-amyloïde et/ou le peptide β-amyloïde. Il s'agit plus préférentiellement de compositions pharmaceutiques destinées au traitement de maladies neurodégénératives comme par exemple la maladie d'Alzheimer.

Un autre objet de la présente invention est l'utilisation des polypeptides mis en évidence par l'invention pour inhiber au moins en partie l'interaction entre une préséniline et le précurseur du peptide β-amyloïde et/ou le peptide β-amyloïde et de manière préférée l'utilisation de ces polypeptides pour l'obtention d'un médicament destiné au traitement des maladies neurodégénératives et notamment la maladie d'Alzheimer.

Pour leur utilisation selon la présente invention, les polypeptides mis en évidence par l'invention d'une part ou toute molécule capable d'inhiber au moins en partie l'interaction entre une préséniline et le précurseur du peptide β-amyloïde et/ou le peptide β-amyloïde, les séquences nucléiques correspondantes d'autre part ou encore des vecteurs sont préférentiellement associés à un ou des véhicules pharmaceutiquement acceptables pour être formulés en vue d'administrations par voie topique, orale, parentérale, intranasale, intraveineuse, intramusculaire, sous-cutanée, intraoculaire, transdermique, etc. De préférence, ils sont utilisés sous une forme orale. La forme injectable peut être néanmoins envisagée et pourra en particulier être formulée avec des solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de vecteur et en particulier de virus utilisées pour l'administration peuvent être adaptées en fonction de différents paramètres, et notamment en fonction du site d'administration considéré (organe, tissu nerveux ou musculaire), du nombre d'injections, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution de virus, et est déterminé par infection d'une culture cellulaire appropriée, et mesure, généralement après 15 jours, du nombre de plages de cellules infectées. Les techniques de détermination du titre pfu d'une solution virale sont bien documentées dans la littérature.

La présente invention offre un moyen efficace pour traiter les maladies pour lesquelles l'interaction entre une préséniline et le précurseur du peptide β-amyloïde et/ou le peptide β-amyloïde est impliquée et de manière préférée pour le traitement des maladies neurodégénératives et notamment la maladie d'Alzheimer.

La présente invention sera plus amplement détaillée à l'aide des exemples ci- dessous considérés de manière descriptive et non limitative.

### Liste des figures

Figure 1 :
   A) Schéma des constructions PS2 tronquées
   B) Expression en cellules COS 1.

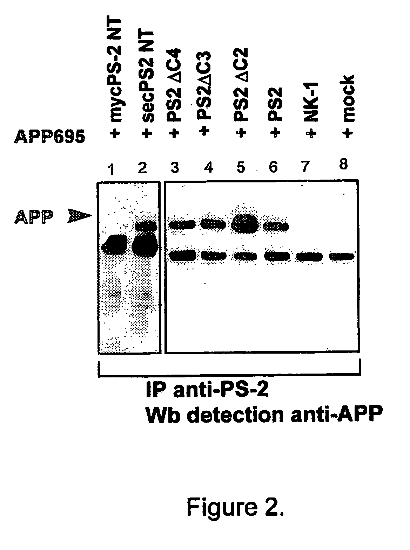

Figure 2 : Interaction des formes tronquées de PS2 avec APP.
Figure 3 :
   A) Interaction du N-term sécrété de PS2 (SecPS2NT) mais pas de sa forme cytoplasmique (myc-PS2NT) et de APP extracellulaire.
   B) Mise en évidence de l'interaction SecPS2NT /APP mais pas de myc -PS2NT /APP en milieu extracellulaire.
Figure 4 : Interaction de PS2 avec les formes tronquées de APP :
   A) Interaction de PS2 avec la forme SPA4CT de l'APP mais pas avec le domaine cytoplasmique de l'APP (MC45F).
   B) Interaction de PS2 avec la forme C100 de l'APP
Figure 5 : Interaction de PS1 et PS1DC2 avec APP et sa forme courte:
   A) Interaction de PS1 avec la forme entière de l'APP et la forme tronquée SPA4CT
   B) Interaction de la forme tronquée de PS1 (PS1 ΔC2) avec l'APP
Figure 6 : Interaction de PS1 et de APP en cellules d'insectes.
   A) Immunoprécipitats anti-Histidine (étiquette sur PS1), révélation APP
   B) Immunoprécipitats anti-PS 1, révélation APP
   C) Immunoprécipitats inverse anti-APP, révélation PS 1
Figure 7 : Interaction de la forme sécrétée de PS2NT avec le peptide Aβ dans le milieu extracellulaire.
Figure 8 : Reconstitution de l'interaction Ap/PS2Nt in vitro sur filtre de nitrocellulose.
   A) Dose-dépendance en fonction de la concentration de PS2Nt
   B) Dose-dépendance en fonction de la concentration de A β.
Figure 9 : Interaction in vitro des formes complètes de PS1 et APP.
   A) Immunoprécipitats anti-Histidine
   B) Immunoprécipitats anti-PS 1
Figure 10 : Déplacement par l'extrémité N-Terminale de PS2 précédée d'un peptide signal (SecPS2NT) de l'intéraction entre PS1 et le fragment SPA4CT
Figure 11 : Interaction de PS2 NT avec le peptide Aβ₁₋₄₂ in vitro :mise en évidence par le test en plaque 96 puits (ELISA)
Figure 12 : Interaction de PS2 NT avec le peptide Aβ₁₋₄₂ mise en évidence par le test de transfert de fluorescence HTRF(homogenous Time-Resolved Fluorescence).
Figure 13 : Le blocage de l'interaction APP/PS1 avec SecPS2NT conduit à l'inhibition de la production de peptide amyloide A β₁₋₄₂ intracellulaire
   A) Mise en évidence que le blocage de l'interaction APP/PS1 avec SecPS2NT conduit à l'inhibition de la production de peptide amyloide Aβ₁₋₄₂ intracellulaire
   B) Contrôle que l'expression de SecPS2NT n'a pas d'influence sur l'expression des différents trangènes
Figure 14 : Détection de l'interaction de PS2 avec l'APP endogène des cellules COS à l'aide du traitement pharmacologique à la lactacystine
Figure 15 : Interaction de PS2 et PS2 NT avec une deuxième région de l'APP, différente du peptide A β.

### Matériels et méthodes

### A/ MATERIELS

### 1. Constructions exprimant les présénilines.

L'obtention de vecteur d'expression (vecteur hote, pcDNA3, *Invitrogen*) en cellules mammiféres des protéines humaines PS1 et PS2 a été décrite précédemment (Pradier et col., 1996). Plusieurs délétions successives par l'extrémité C-term de PS2 ont été générées (voir Fig1A). Les numérotations sont faites à partir du codon d'initiation de PS2 comme position 1.

Le fragment de restriction HindIII du vecteur PS2 (du 5' non-codant, position -55, au site interne à la position 1080) a été purifié et mis en ligation avec le vecteur pcDNA3 linéarisé par HindIII et traité à la phosphatase alkaline. La PS2 tronquée ainsi produite (PS2ΔC1) s'étend de l'extrémité N-terminale au résidu 361 plus 7 résidus apportés par l'extrémité 3' et comprend donc les six premiers domaines transmembranaires et une large partie de la boucle hydrophile (Fig1A).

PS2ΔC2 a été construit par digestion du plasmide PS2 par PstI (site interne à position 679) et ligation avec le fragment PstI correspondant à la partie 3'non-codante du vecteur PS2. La protéine tronquée PS2ΔC2 s'étend de la position 1 au résidu 228 de PS2 plus 18 résidus supplémentaires apportés par l'extrémité 3'. Elle inclut les quatre premiers domaines transmembranaires de PS2.

Une construction similaire a été effectuée pour PS2 portant la mutation N141I, PS2 ΔC2*.

Le fragment de restriction HindIII (-55)/MscI(590) de la construction PS2ΔC2 a ensuite été cloné dans le vecteur pcDNA3 traité par HindIII et dont l'extrémité Apal a été rendue à bout franc. Cette construction PS2ΔC3 s'étend de l'extrémité N-term au résidu 198 plus 2 résidus supplémentaires comprennant donc les trois premiers domaines transmembranaires de PS2.

Le fragment de restriction de PS2ΔC2, HindIII (-55)/NcoI(504) rendu à bout francs en son extrémité Ncol par traitement au fragment Klenow de l'ADN polymérase a été recloné dans le même vecteur pcDNA3 HindIII/(ApaI bout franc) pour construire PS2ΔC4 s'étendant jusqu'au résidu 168 de PS2 plus 3 résidus supplémentaires.

La construction de l'extrémité N-terminale hydrophile de PS2 a été obtenue par amplification de la séquence PS2 avec les oligonucléotides ext5':
5'-CGGAATTCATCGATTCCACC ATGCTCACATTCATGGCC-3' (SEQ ID4) (chevauchant l'ATG initial, en gras, et introduisant un site de restriction EcoRI, souligné) et ext3':
5'-CCGCTCGAGTCATTGTCGACCATGCTTCGCTCCGTATTTGAGG-3' (SEQ ID5) (introduisant un codon stop aprés le résidu 90 de PS2 ainsi qu'un site de restriction XhoI, souligné).

Après clonage dans le vecteur pCRII par la méthode TA cloning (InVitrogen), la conformité du fragment de PCR a été vérifié par séquençage. Ce fragment (EcoRI/XhoI) a ensuite été introduit dans un vecteur pcDNA3 en phase avec une séquence correspondant à l'épitope myc à son extrémité N-terminale: mycPS2Nter.

Pour ne pas préjuger de la topologie de PS2, le même fragment Nter a été recloné dans le vecteur pSectagB, en phase avec la séquence du peptide signal de IgkB pour diriger la sécrétion de la protéine pS2Nter: SecPS2-Nter.

L'extrémité C-term de PS2 a été construite de façon similaire à l'aide du fragment de restriction HindIII (1080)/PstI(en 3'non-codant) recloné dans le vecteur pSecTagB HindIII/PstI, SecPS2Cter s'étendant du résidu 361 à l'extrémité C-terminale, ou dans le vecteur pcDNA3myc en phase avec l'épitope myc.

De même, une construction tronquée de PS1 a été obtenue. Le vecteur pcDNA3-PS1 a été digéré par PflmI (site à la position 636 de la séquence nucléique codante de PS1) et Xhol dans le 3'non codant de la séquence de PS1. Ces sites ont été transformés à bout franc par traitement à la T4 ADN polyméase. Le fragment vecteur, purifié sur gel d'agarose, a été religé sur lui-même pour fournir un vecteur d'expression d'une PS1 tronquée s'étendant du N-ter de PS1 jusqu'au résidu Ile213 (après le 5eme domaine transmembranaire) plus 12 résidus supplémentaires. Cette construction correspond à la chimère ΔC2 et est appelée PS1 ΔC2.

### 2.Constructions exprimant l'APP.

### 2.1 Constructions APP

Les différentes constructions APP complet (isoforme 695) et SPA4CT (les 100 derniers résidus de l'APP (acide aminé 597 à 695) précédés d'un peptide signal pour insertion à la membrane) ont été décites précédemment (Dyrks et coll., 1993). Les vecteurs, pour l'expression de C100 et du domaine cytoplasmique de l'APP, ont été obtenus de la manière suivante : les cDNA correspondants ont été obtenus par amplification enzymatique de l'ADN (PCR (Polymerase Chain Reaction)) en utilisant, comme amorce de synthèse, les oligonucléotides suivants: pour le C100 : les oligonucléotides 8172 et 8181; pour le domaine cytoplasmique de l'APP: les oligonucléotides 8171 et 8181.
**Oligo 8172 5' CAAAGATCTGATGCAGAATTCCGACAT 3'(SEQ ID6)** contenant:
- un site de reconnaissance pour l'enzyme de restriction BgIII (souligné)
- la séquence codante pour les acides aminés 597-602 de l'APP (en gras) [numérotation APP de 695 acides aminés]
**Oligo 8181 5' CAAGCGGCCGCTCATCC*GTTGTCATCGTCGTCCT***
*TGTAGTC*TCC**GTTCTGCATCTGCTC** 3' (SEQID7) contenant :
- un site de reconnaissance pour l'enzyme de restriction Notl (souligné)
- la séquence complémentaire à la séquence codante pour les acides aminés 691-695 de l'APP (en gras) [numérotation APP de 695 acides aminés]
- la séquence complémentaire à la séquence Asp-Tyr-Asp-Asp-Asp-Asp-Lys correspondant à l'épitope FLAG (en italique).
**Oligo 8171 5'CAAAGATCTAAGAAACAGTACACATCC 3'(SEQ ID8),** contenant:
- un site de reconnaissance pour l'enzyme de restriction BglII (souligné)
- la séquence codante pour les acides aminés 650-655 de l'APP (en gras) [numérotation APP de 695 acides aminés]

Les produits de l'amplification enzymatique de l'ADN ont été clonés dans le vecteur pCRII. La séquence nucléotidique a été vérifiée par la méthode des terminateurs spécifiques d'ADN.

Les cDNA sont ensuite introduits par ligation dans le plasmide d'expression dérivé du plasmide pSV2 et contenant, dans le même cadre de lecture, un épitope MYC.

### 2.2.Construction des formes solubles de l'APP: a-sAPP et β-sAPP.

Les ADNc correspondants aux formes sécrétées de l'APP se terminant aux sites α- et β- de clivage ont été obtenus par PCR.

L'oligonucléotide 1:
**5'-ccatcgatggctaCATCTTCACTTCAGAG-3' (SEQ ID 9)** introduit:
- un codon stop (séquence complémentaire inverse soulignée) après la position 1788 de l'APP correspondant au site de clivage β.
- et un site de restriction ClaI.

L'oligonucléotide 2:
5'- **ccatcgatggctaTTTTTGATGATGAACTTC** -3' (SEQ ID 10) introduit:
- un codon stop (séquence complémentaire inverse soulignée) après la position 1836 de l'APP correspondant au site de clivage α.
- et un site de restriction ClaI.

L'oligonucléotide 3:
5'- **CCGTGGAGCTCCTCCCG** -3'(SEQ ID 11), commun pour les deux formes, correspond à la région 1583 à 1600 de l'APP incluant le site de restriction interne de l'APP SacI (souligné).

L'ADNc de l'APP a été amplifié par PCR en utilisant les paires oligo3-oligol et oligo3-oligo2 pour β-sAPP et α-sAPP respectivement. Les produits d'amplifications ont été sous-clonés comme précédemment en pCRII et les séquences vérifiées par séquençage. Pour chacun, le fragment de restriction SacI-ClaI ont été purifiés et reclonés dans le vecteur d'expression APP (voir ci-dessus) digéré lui-aussi par SacI-ClaI pour remplacer la partie C-terminale de l'APP par les fragments C-terminaux de β-sAPP et α-sAPP respectivement et reconstituer les protéines complètes.

### 3. Constructions baculovirus.

L'obtention de vecteur de transfert pour baculovirus codant pour la protéine humaine PS1 a été réalisé à partir de vecteur d'expression pour cellules de mammifères (Pradier et col, 1996). L'ADNc codant pour la protéine PS1 a été extrait par une digestion par les enzymes de restriction Xhol et NotI, puis cloné dans le plasmide de transfert pAcHTLB (protéine de fusion 6Histidines) et pAcSG2 (protéine native). L'obtention de baculovius recombinants s'effectue selon le protocole du fournisseur (Pharmingen) et consiste à cotransfecter 2 X 10⁶ cellules d'insectes (sf9) avec 1 µg de plasmide de transfert contenant le gène d'intérêt et 0.5 µg d'ADN viral (Baculogold). Après 5 jours à 27°C, les cellules sont grattées, puis centrifugées, le surnageant est utilisé comme stock viral pour l'amplification et la détermination du titre viral, l'expression de la protéine est visualisée par western blot sur le culot cellulaire.

L'obtention du baculovirus exprimant l'APP Humain (695) a été décrite précédemment (Essalmani et col, 1996).

Pour l'étude de l'expression de PS1 et d'APP, les cellules sf9 sont coinfectées à une M.O.I de 2 par des baculovirus exprimant l'APP humain (695), la protéine humaine Présiniline 1 (PS1), ou PS1 avec un tag 6 histidine en N terminal (6HisPS1), ou la protéine de contrôle de pseudomas putrida XylE avec un tag 6 histidine en N terminal (6HisXylE), puis solubilisées par un tampon Tris 10mM, NaCl 130mM, Triton X100 1%, NP 40 1%, pH 7.5.

Les protéines solubilisées sont immunoprécipitées par un anticorps antiHistidine (A), antiPS1 (1805)(B), ou antiAPP (22C11) (C). La présence de l'APP ou de PS1 a été révèlée par western blot avec l'anticorps antiAPP aCT43(A), 22C11(B), ou l'anticorps antiPS1 95/23(C).

Les fractions solubilisées contenant l'APP, PS1 ou 6HisPS1 sont mélangées, puis immunoprécipitées en présence d'anticorps anti Histidines ou antiPS1 pendant une nuit à 4°C. La coimmunoprécipitation de l'APP est révélée après western blot avec les anticorps αCT43 ou 22C 11.

### 4. Les plasmides

Les plasmides utilisés pour l'invention sont les suivants:
- pcDNA est un plasmide commercial (InVitrogen) utilisé pour le clonage et l'expression en cellules mammiféresdes séquences PS1 et PS2 et de leur formes tronquées.
- pCRII est un plasmide commercial (InVitrogen), utilisé pour le clonage de fragments de PCR
- pSecTagB est un plasmide commercial (InVitrogen), utilisé pour le clonage et l'expression en cellules mammiféres d'ADNc auxquels sont rajoutés le signal de sécrétion (peptide signal Ig κ).
- pSV2 est un plasmide commercial (Pharmacia), utilisé pour le clonage et l'expression en cellules mammiféres d'ADNc.
- pAcHTLB est un plasmide commercial (Pharmingen), pour l'insertion d'un épitope (His)6 à des ADNc et la recombinaison homologue avec des baculovirus.
- pAcSG2 est un plasmide commercial (Pharmingen), pour la recombinaison homologue avec des baculovirus.
- pET29a est un plasmide commercial (Novagene), pour l'expression d'ADNc en bactéries

### B/ METHODES

### 1. Transfection de cellules

La méthode établie pour les cellules COS1 ou les cellules CHO, consiste à utiliser un lipofectant dans un ratio de 1 pour 8 (poids/poids) par rapport à l'ADN et un peptide synthétique H1 (séquence: KTPKKAKKPKTPKKAKKP) au même ratio afin d'optimiser la compaction de l'ADN et l'efficacité de transfection. Cette méthode repose notamment sur la neutralisation des charges des phosphates de l'ADN par les charges positives du lipofectant.

Les cellules COS1 sont cultivées en incubateur à 37°C, 95% d'humidité et 5 % de CO₂ dans le milieu DMEM (Dulbecco's Modified Eagle's Medium) contenant 4.5 g/l glucose (Gibco-BRL) supplementé avec 3 % de L-Glutamine, 1% de pénicilline-streptomycine et 10 % de Sérum de Veau Foetal.

La veille de la transfection, les cellules sont ensemencées à une densité de 2.5 10⁶ cellules par boite de 100 mm. Le jour de la transfection les cellules sont rincées 2 fois par du PBS (Phosphate Buffer Saline) et une fois en OptiMEM (composition brevetée; Gibco-BRL) pour une habituation d'au moins 15 minutes en incubateur.

Par équivalent-boite de 100 mm, 8 µg d'ADN plasmidique au total sont ajoutés à 300 µl d'OptiMEM et 64 µg de peptide H1. Après avoir vortexé vigoureusement pendant 10 secondes, on attend 5 minutes et la lipofectamine (32 µl, soit 64 µg) diluée dans 300 µl d'OptiMEM est ajoutée au mélange précédent. L'ensemble est une nouvelle fois vortexé vigoureusement puis laissé 30 minutes reposer. Cinq millilitres d'OptiMEM sont ajoutés par tube et le mélange vortexé est placé sur les cellules (dont le milieu a été au préalable aspiré). Les cellules sont alors placées en incubateur pendant 4 heures, au terme desquelles le mélange est remplacé par du milieu complet.

### 2.Lyse des cellules et dosage des protéines

Les cellules sont le plus souvent lysées 48 heures après la transfection (au maximum habituel d'expression). Le tampon de lyse contient 10 mM de Tris pH 7.5, 1 mM d'EDTA, 1 % de Triton X100, 1 % de NP40 et un cocktail d'inhibiteurs de protéases (Complete©, Boehringer-Mannheim). Pour chaque plaque, après un rinçage au PBS, 800 µl du tampon froid sont ajoutés. Les lysats subissent alors une sonication suivie d'une agitation au barreau magnétique à 4°C pendant une nuit. Une centrifugation de 30 minutes à 15000 tr/mn sépare le culot du surnageant. Les protéines solubles sont alors dosées selon le kit BCA (Pierce) afin de pouvoir normaliser les expériences suivantes.

### 3.Immunoprécipitations

Les anticorps dirigés contre le peptide du N-term de PS2, 95041, (Blanchard et coli., 1997) et contre les vingt premiers acides aminés de PS1 (Duff et coll., 1996) ont été obtenus chez le lapin par immunisation avec des peptides synthétiques. Pour l'immuno-précipitation, 100 µg de protéines sont dilués dans 400 µl de RIPA modifié (150 mM NaCl, 50 mM Tris pH 8.0, 1 % Triton X100 v/v, 1% NP40 v/v). Trente microlitres de suspension de protéine A Sépharose (0.1 % m/v en solution de PBS) et 3 µl d'anticorps sont ajoutés. Les suspensions sont mélangées doucement sur un agitateur rotatif à 4°C pendant une nuit. Le complexe de protéine A Sépharose est lavé 3 fois avec 0.5 ml de RIPA modifié et une fois avec 0.5 ml de tampon de lavage « Wash C » (10 mM Tris pH 7.5).

### 4.Immunotransfert

Les échantillons (lysats de cellules) sont dénaturés dans un volume égal de tampon de dépôt (125 mM Tris pH 6.8, 4% m/v SDS, 20 % glycérol, 0.02 % Bromophénol Blue, 50 mM Dithiothreitol) à 95°C pendant 5 minutes. Pour l'analyse de l'expression des présénilines, les échantillons sont dénaturés en présence de 8 M urée et à 37 °C afin d'éviter l'agrégation propre aux présénilines à 95 °C.

Les échantillons sont déposés sur des gels Tris-Glycine (Novex), avec un pourcentage d'acrylamide différent selon le poids moléculaire à discriminer. Un marqueur de Poids Moléculaire est également déposé (Broad Range, BioRad). La migration a lieu pendant environ 2 heures à 100 Volts constants dans du tampon SDS 1X final (Novex). Le gel est ensuite transféré sur une membrane de nitrocellulose ou de PVDF (Tampon de transfert 1X final (Novex) avec 10 % de méthanol) pendant 2 heures à 150 mA constants.

Après transfert, la membrane est bloquée pendant 2 heures à température ambiante dans 50 ml de PBS-T (PBS avec 0.5 % Tween) contenant 2 % de lait écrémé (Merck). L'anticorps primaire (dilué à la concentration optimale de l'ordre du 1/1000e au 1/5000e, dans du PBS-T avec ou sans 2 % de lait écrémé) est laissé sur une nuit à 4 °C. Après un bref rinçage en PBS-T, la membrane est incubée 45 minutes en présence du deuxième anticorps (IgG anti-souris ou anti-lapin selon le cas, couplé à la peroxydase de Raifort) dilué au 1/5000^{e} dans un tampon dit «ECL» (Tris 20 mM, NaCl 150 mM, Tween 0.1 %).

La membrane est alors rincée 4 fois 15 minutes dans le tampon «ECL». Elle peut être révélée par le réactif ECL (Amersham) constitué de 2 tampons à mélanger extemporanément en volume égal. Différentes expositions d'un film photographique (Hyperfilm ECL ; Amersham) sont effectuées, suivies d'un développement.

### 5.Fixation in vitro de PS2 NT avec le peptide Aβ Amyloïd

### 5.1 Production de la protéine recombinante PS2 NT en bactérie

Pour la création d'un vecteur d'expression bactérien de PS2NT (amino acides 1 à 87), l'ADNc de PS2 a été amplifié par PCR avec les oligonucléotides 3':(CCGCTCGAGTCATTGTCGACCATGCTTCGCTCCGTATTTGAGG) et 5' (CCGGAATTCATCGATTCCACCATGCTCACATTCATGGCC). Le fragment résultant a été cloné dans pCRII et la séquence confirmée. Ce fragment a ensuite été sous-cloné dans le vecteur pET29a (Novagene) en phase avec la séquence de l'étiquette S-tag. La protéine a été produite en bactérie BL21 Après induction à l'IPTG pour 5h, les bactéries ont été récupérées par centrifugation (10 min à 6000tours/mn) et le culot cellulaire dissout en tampon RIPA (volume calculé en multipliant la DO de la culture après induction par le volume de culture divisé par 23). Les bactéries ont été lysées par sonication et le lysat centrifugé à 13000 tr/min pendant 20 min à 4°C. Le surnageant (extrait total) a été utilisé pour les études de fixation.

La protéine recombinante PS2NT a aussi été purifiée de l'extrait total sur colonne de Nickel (étiquette poly-His apportée dans le vecteur pET29a) comme décrit par le fournisseur (Novagene).

### 5.2 Test de fixation PS2NT/Aβ42 sur membrane de nitrocellulose

Le peptide Aβ synthétique (en solution) a été déposé sur membrane de nitrocellulose (Schleicher and Schuell) en utilisant un appareil dot-blot 96 puits. Après dépôt, le filtre a été bloqué (envers les sites non-spécifiques de fixation de protéines) avec le réactif de bloc gélatine (Novagen) dilué au 10^{eme} en TBST. Après bloquage, le filtre a été replacé sur l'appareil de dot-blot et l'extrait bactérien PS2NT ajouté dans les puits pour une incubation de 2h à température ambiante. Comme contrôle, un extrait bactérien contenant le plasmide vide pET29 a été utilisé sur des puits en duplicat. Le filtre a ensuite été lavé une fois avec du tampon RIPA, puis retiré de l'appareil et lavé trois fois avec du PBST (15min pour chaque lavage). La détection de l'étiquette S-tag a ensuite été effectuée comme prescrit par le fournisseur (Novagen) avec un substrat colorimétrique. La quantification de la réaction colorimétrique (précipitat) a été effectuée par scanning optique du filtre et quantification de l'intensité dans chaque puit par le logiciel Tina 2.1 (Raytest).

### 5.3 Test d'interaction PS2NT/A β42 en format ELISA

Le peptide Aβ (1-40 et 1-42) synthétique (100µl, 2µg/ml) est incubé sur la nuit en plaques 96 puits pour fixation sur le plastique. Les plaques sont rincées deux fois avec du PBS et les sites de fixation non-spécifiques sont saturés par incubation avec 5% (p/v) d'Albumine de Serum de Boeuf en PBS. La protéine recombinante purifiée (selon le protocole décrit en 5.1), PS2NT, diluée en tampon (25mM Tris/HCl, pH 7.5, 0.5% Triton X-100, 0.5% NP40) est ajoutée et incubée pendant 4h, à température ambiante. Après deux rinçages en PBS-Tween 0.5%, la protéine PS2NT retenue sur la plaque (en interaction avec le peptide Aβ) est révélée par incubation avec la protéine fixatrice du S-Tag couplée à l'alkaline phosphatase comme précédemment. La détection du signal est effectuée dans un spectrophotomètre à 450 nm.

### 5.4 Test d'interaction PS2NT/Aβ1-42 en format HTRF (Homogeneous Time-Resolved Fluorescence).

La protéine purifiée PS2NT (produite selon le protocole décrit en 5.1) a été marquée à l'aide du fluorophore cryptate d'Europium (PS2NT-K). Les peptides Aβ₁₋₄₀ et Aβ₁₋₄₂ ont été synthétisés avec une biotine et un bras écarteur de 3 βAlanines (ou de 3 lysines) à leur extrémité N-terminale (en amont de la position 1 des peptides Aβ) et deux Arginines (R) en leur extrémité C-terminale pour faciliter la synthèse, peptides biot-3K-Aβ40_{RR} et biot-3K-Aβ42_{RR}.

La réaction d'interaction de PS2NT-K avec biot-Aβ40 ou biot-Aβ42 est effectuée dans un tampon 10mM HEPES, pH=7.2, contenant 150mM NaCl, 3.4 mM EDTA et 3 mM CHAPS (détergent). La protéine PS2NT marquée (conc. finale 6nM, soit 40 µl de solution initiale à 15nM) est incubée avec le peptide biot-Aβ40 ou biot-Aβ42 (conc. finale 2 µM, soit 40 µl de solution initiale à 5 µM et 20 µL de tampon) pendant 10 min. suivi par l'addition de streptavidine marquée à la XL665 (XL665 est une allophycocyanine crosslinkée, CisBio International) à la concentration de 8µg/ml (soit 100µl de solution initiale à 16 µg/ml) dans un tampon HEPES 100mM pH=7.0 contenant 400mM KF, 133mM EDTA et 1g/l BSA. La réaction est incubée, soit 4h à température ambiante, soit 24 h à 4°C et les plaques sont lues sur un compteur Packard Discovery qui mesure d'une part l'émission du cryptate d'Europium à 620nm après excitation à 337nm et d'autre part l'émission de la XL665 à 665nm après transfer de la fluorescence à 620nm par le cryptate d'Europium sur la XL665. La formation du complexe XL665-streptavidine/biot-Aβ42/ PS2NT-cryptate conduit à un transfert de fluorescence du cryptate vers la XL665 qui est mesuré à 665 nm par le compteur. En l'absence de formation d'un complexe XL665-streptavidine/biot-Aβ42/ PS2NT-cryptate, le cryptate d'Europium fluoresce à 620nm.

### EXEMPLES

### Exemple 1. Interaction entre APP et PS2 et cartographie de la zone d'interaction sur PS2.

Cet exemple a pour but de déterminer la zone d'interaction sur PS2 et de mettre en évidence une interaction entre ladite région et l'APP.

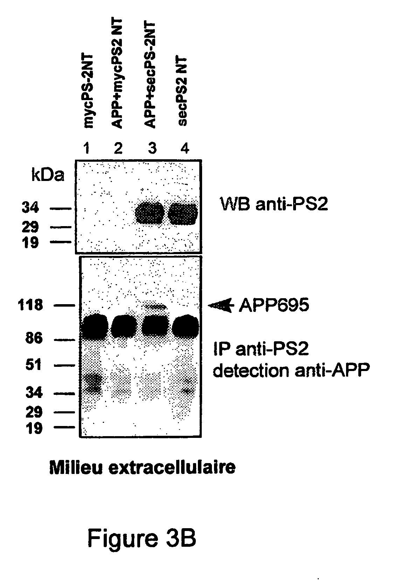

L'interaction entre les protéines APP et PS2 en cellules mammiféres est exemplifié dans la figure 2. Le lysat de cellules COS transfectées avec PS2 et APP est soumis à une immunoprécipitation avec un anticorps dirigé contre le N-term de PS2 ( 95041, Blanchard et col., 1997). L'immunoprecipitat est ensuite analysé par immunotransfert avec un anticorps contre l'APP. L'APP est clairement détecté dans les immunoprécipitats des cellules cotransfectées avec APP et PS2 mais pas en l'absence de PS2 (Fig2., piste 6 par rapport à piste 7) comme précedemment décrit (Weidemann et col., 1997). Pour cartographier la zone d'interaction entre ces deux protéines, plusieurs formes tronquées de PS2 ont été construites. Afin de conserver la topologie membranaire de PS2 déterminée en général par la partie N-term des protéines membranaires, des tronquations progressives de l'extrémité C-term de PS2 ont été produites se terminant après différents domaines transmembranaires TM6 (PS2ΔC1), TM4 (PS2ΔC2), TM3 (PS2ΔC3) et TM2 (PS2ΔC4), schéma Fig1A. L'extrémité N-term hydrophile (87 résidus) de PS2 a aussi été construite sous forme cytoplasmique (séquence native) ou sous forme sécrétée par l'insertion du peptide signal de la chaine Igk. L'expression de ces différentes formes est exemplifiée figure 1B, révélée à l'aide de l'anticorps anti-PS2 (95041). Les constructions possédant des domaines hydrophobes présentent en plus des bandes correspondant aux formes monomériques au poids moléculaires attendus (formant ici des doublets rapprochés), des formes dimériques et des aggrégats de haut poids moléculaires typiques de PS2 (Fig 1B, pistes 3-5). En particulier pour PS2 complète, seuls ces aggrégats sont détectables dans cette figure tandis que la forme monomérique n'est pas détectable (piste 6). Les deux constructions du N-term hydrophille de PS2: mycPS2Nt et SecPS2Nt donnent lieu aux bandes aux poids moléculaires attendus ( Fig1B, pistes 1 et 2.). La construction SecPS2Nt est également sécrétée dans le milieu extracellulaire (Fig 3B, piste 2) tandis que la construction mycPS2Nt est, elle, cytoplasmique.

Ces constructions ont été cotransfectées individuellement avec l'APP. La fraction détergent-soluble des lysats cellulaires a été immunoprécipitée avec l'anticorps dirigé contre le N-term de PS2 et ces immunoprécipitats analysés par immunoblots. Comme avec la PS2 complète, l'APP est détectable dans les immunoprécipitats avec toutes les formes tronquées de PS2, PS2ΔC2 à PS2ΔC4 (Fig2, pistes 3-5) démontrant l'interaction entre APP et les formes contenant le N-term de PS2. Cette interaction avec l'APP est conservée avec la construction N-term de PS2 sous sa forme sécrétée (Fig2, piste 2) démontrant que l'ancrage de PS2Nt dans la membrane lipidique n'est pas nécessaire pour cette interaction. Par opposition, la forme cytoplasmique mycPS2NT n'interagit pas avec APP (Fig2,piste 1).

L'expérience inverse d'immunoprécipitation par un anticorps anti-APP et de la détection par l'anticorps N-term de PS2 a permis de confirmer l'interaction entre l'APP et SecPS2Nt dans des conditions expérimentales différentes .

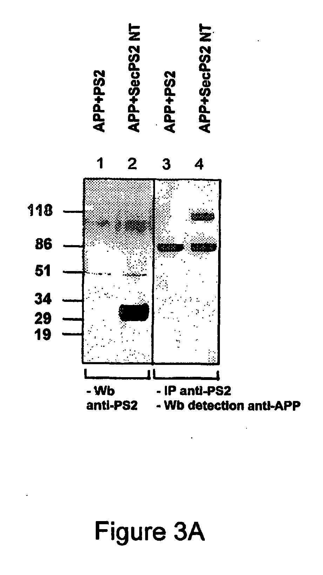

Dans le milieu de culture des cellules cotransfectées avec APP et SecPS2Nt, une interaction entre ces deux protéine est également démontrée par coimmunoprécipitation (Fig3A, piste4 et Fig3B, piste3). La forme mycPS2NT n'interagit pas avec APP dans le milieu (Fig3B, piste2). La présence de cette interaction dans le milieu, démontre que le complexe APP/PS2Nt est relativement stable au cours du processus de sécrétion.

### Exemple 2. Interaction entre APP et PS2 et cartographie de la zone d'interaction sur APP.

Cet exemple a pour but de déterminer la zone d'interaction en ce sur l'APP et de mettre en évidence une interaction entre ladite région et la préséniline 2.

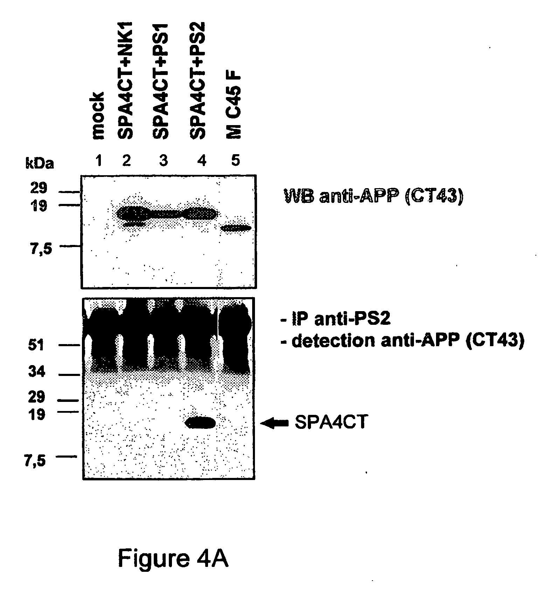

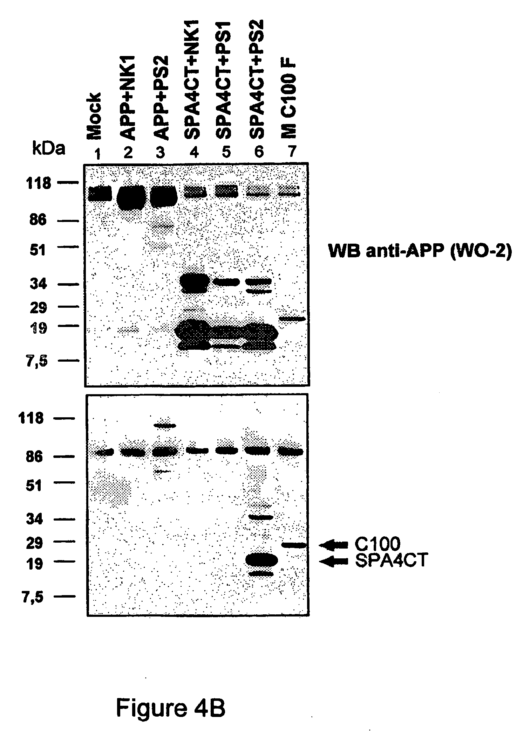

A cet effet, des formes tronquées de l'APP ont été utilisées pour délimiter la zone d'interaction sur l'APP. Une construction comportant les 100 derniers résidus de l'APP sous le contrôle ou non d'un peptide de sécrétion (SPA4CT et C100, Dyrks et col.,1993) et une construction comportant seulement le domaine cytoplasmique (les 45 derniers résidus de l'APP) ont été utilisées et leur présence détectée à l'aide d'un anticorps dirigé contre le domaine cytoplasmique de l'APP (αCT43, Stephens et Austen, 1996). Dans les cellules cotransfectées avec PS2, une interaction de SPA4CT mais pas du domaine cytoplasmique de l'APP avec PS2 a pu être mis en évidence (Fig 4A, comparer pistes 4 et 5). L'interaction de PS2 avec la construction C100 (sans signal de sécrétion) a aussi pu être démontrée (Fig4B, piste 7). Même en associant le domaine cytoplasmique de l'APP à la membrane dans une construction chimérique avec le récepteur alpha de l'IL2, aucune interaction avec PS2 n'a pu être observée. Cet exemple démontre qu'il existe une interaction avec SPA4CT (résidus 597 à 695 de l'APP) mais pas avec le domaine cytoplasmique (résidus 651 à 695) indiquant donc que sur l'APP, la région du Aβ (résidus 597 à 637) et le reste du segment transmembranaire (jusqu'au résidu 650) sont suffisantes pour l'interaction avec PS2.

### Exemple 3. Interaction de PS1 avec APP et cartographie initiale.

Cet exemple a pour but de déterminer la zone d'interaction sur PS 1 et de valider l'interaction entre ladite région et l'APP.

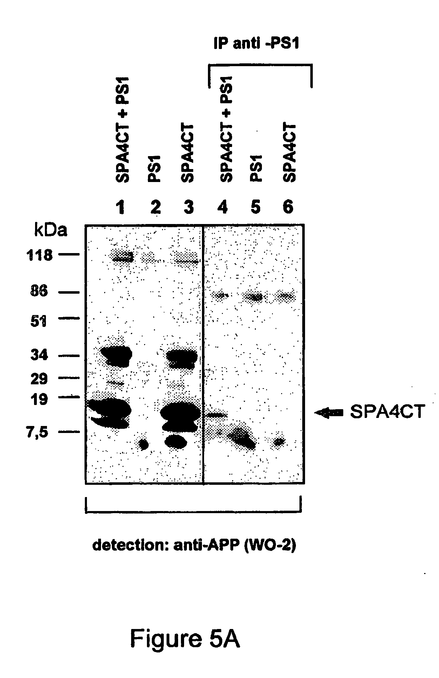

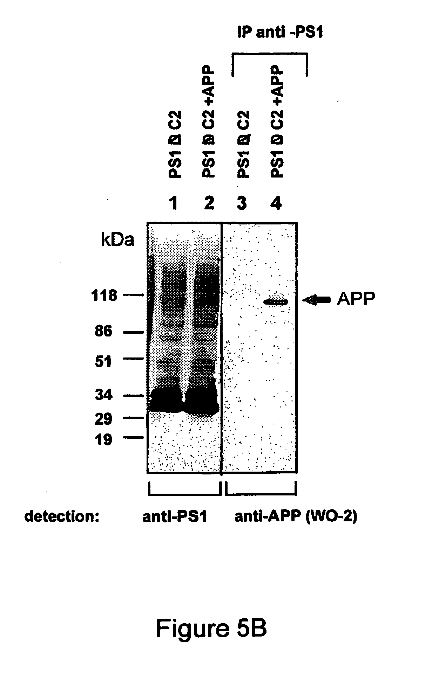

Par analogie aux résultats obtenus pour PS2 (exemples 1 et 2), l'étude de l'interaction de PS1 avec APP a été réalisée dans le même système cellulaire COS1. Après coimmunoprécipitation avec un anticorps dirigé contre les 20 derniers acides aminés de PS1 (Duff et col., 1996), le SPA4CT, le fragment C-terminal de l'APP, a pu être détecté dans les précipitats (Fig. 5A, piste4 ). L'APP aussi interagit avec PS1. De même, la forme tronquée de PS1, PS2ΔC2(1-213), interagit avec APP (Fig5B. piste4). Ces premières données nous permettent d'envisager que les régions d'interaction entre APP et PS1 doivent être voisines de celles exemplifiées précédemment avec PS2.

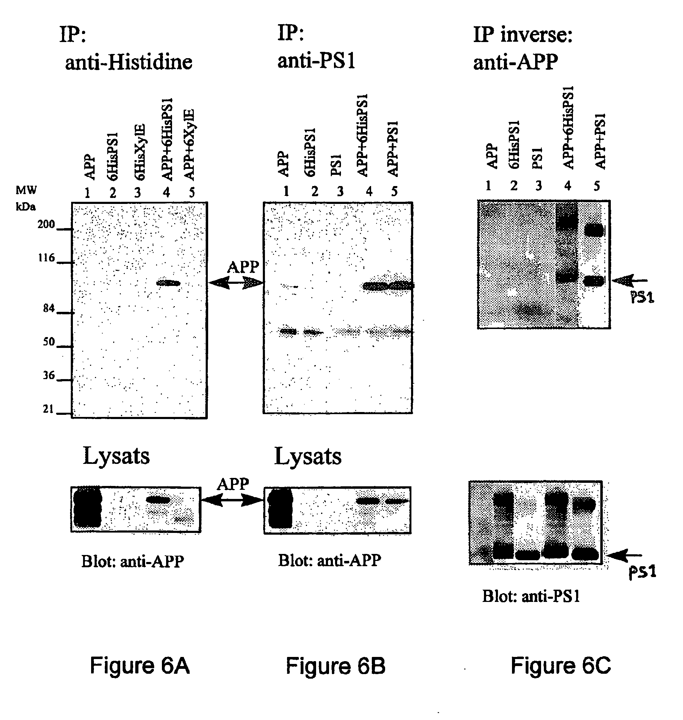

Pour vérifier la validité et la généralité de cette interaction PS1/APP, un système cellulaire différent a été utilisé, dans lequel les cellules d'insectes ont été infectées par des baculovirus recombinants exprimant la PS1 avec ou sans étiquette His6 et l'APP ( c.f. Materiels et methodes). L'étude des lysats cellulaires a permis de détecter l'APP dans les immunoprécipitats antiHis6 (pour la PS1-His6, Fig6A, piste4) ou antiPS1 (pour la PS1 avec ou sans His6, Fig6B, pistes 4 et 5) lorsque les cellules sont coinfectées avec les deux types de virus recombinants mais pas lorsqu'une seule des protéines est exprimée (pistes 1,2 et 3 correspondantes). Inversement, dans les immunoprécipitats anti-APP, les protéines PS1-His6 et PS1 sont détectables (Fig6C, pistes 4 et 5) pour les doubles infections. Cette expérience permet de confirmer l'interaction dans le sens inverse avec des anticorps différents.

### Exemple 4. Interaction de Aβ et PS2 en cellules

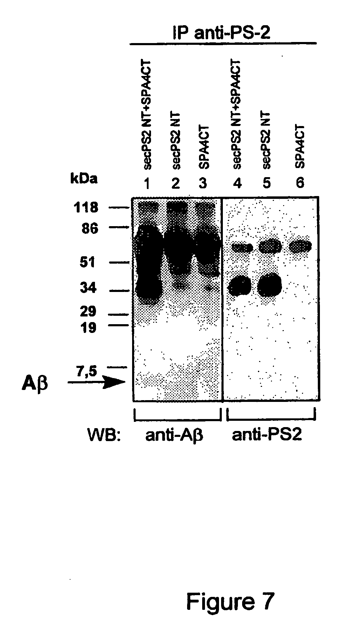

Etant donné que la région d'interaction entre l'APP et la PS2 implique sur l'APP, une région incluant le peptide amyloide (de 595 à 635) et sur la PS2, son domaine N-term hydrophile, il est démontré dans cet exemple que cette dernière interagissait directement avec le peptide amyloïde (Aβ) produit par des cellules. L'expression de SPA4CT (correspondant aux 100 derniers résidus de l'APP précédés d'un petide signal) en cellules COS conduit à une forte production du peptide amyloide, en partie car SPA4CT est considéré comme le précurseur biologique du Aβ. Les cellules COS ont été transfectées avec SPA4CT seul, SPA4CT et SecPS2Nt ou avec SecPS2Nt seul. Les milieux extracellulaires correspondant ont été immunoprécipités avec l'anticorps antiPS2 et le peptide Aβ a été détecté grâce à l'anticorps spécifique W02 (Nida et al. (1996) J. Biol. Chem.271, 32908-914)(Fig7). Le peptide Aβ est identifié seulement pour les cellules cotransfectées avec SecPS2Nt et SPA4CT comme une bande de faible intensité (Fig7, piste 1) mais pas avec les contrôles individuels (Pistes 2 et 3). Par ailleurs, une bande supplémentaire à approximativement 40 kDa est aussi détectée de façon spécifique pour les cellules doublement transfectées. Après lavage du filtre et détection avec l'anticoprs PS2, il apparait qu'une bande au même poids moléculaire est également PS2-immunoréactive (Fig7, piste 4). Cette bande est aussi présente pour les cellules transfectées avec SecPS2Nt comme attendu. Ainsi, dans les cellules doublement transfectées, cette bande représente un complexe SDS-stable entre SecPS2Nt et le Aβ, pouvant confirmer l'interaction entre ces deux entités. La faible différence de masse apportée par le peptide Aβ (4kDa) expliquerait qu'il n'y ait pas de différence de taille détectable avec les cellules transfectées avec SecPS2Nt seulement. Les résultats de ces expérinces permettent de conclure que la forme sécrétée de PS2 (secPS2Nt) interagit in vitro avec le peptide Aβ (résidus 597-637 de l'APP695).

### Exemple 5. Reconstitution de l'interaction de Aβ et PS2Nt en test in vitro sur membranes de nitrocellulose.

Cet exemple a pour but de démontrer la reconstitution de l'interaction PS2Nt-APP(A β) in vitro.

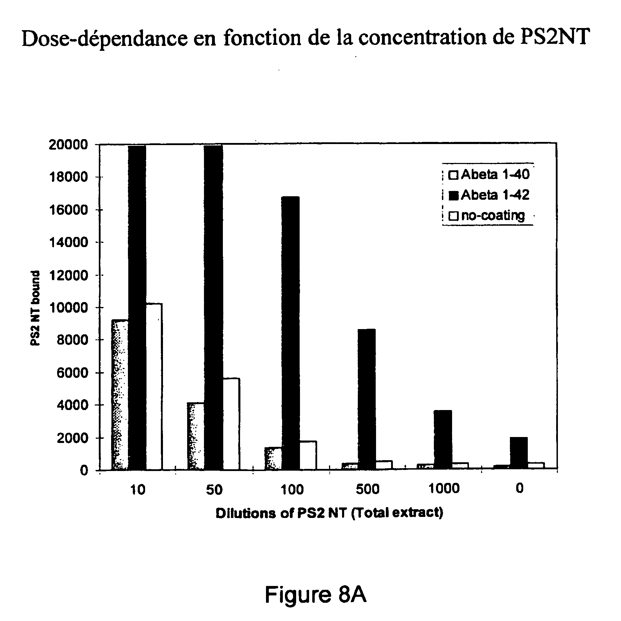

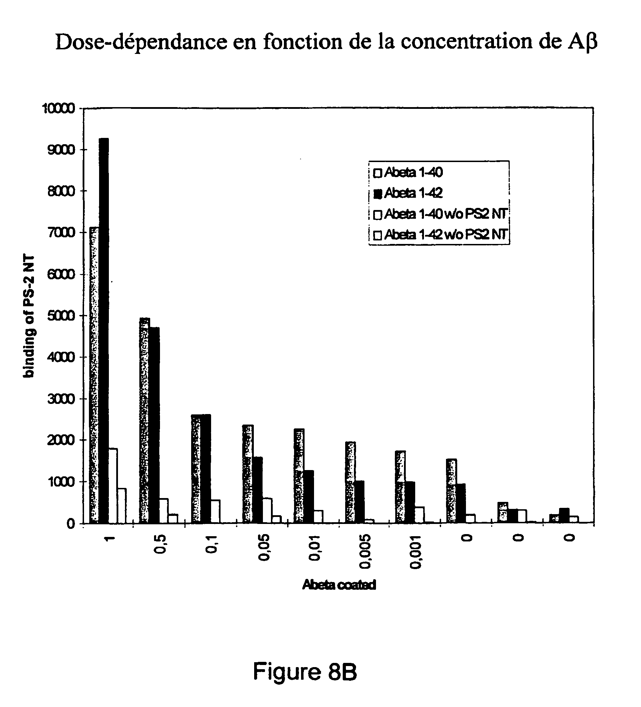

Pour confirmer l'interaction entre le peptide Aβ et PS2Nt (extrémité N-terminale de PS2), un test de fixation in vitro a été développé. Une protéine de fusion PS2Nt portant le peptide étiquette/marqueur S (S-tag) à son extrémité N-terminale a été construite et exprimée en bactérie. Les peptides Aβ₁₋₄₀ et Aβ₁₋₄₂ ont été déposés sur membranes de nitrocellulose qui ont été incubées en présence d'un extrait bactérien exprimant la protéine PS2Nt. Le S-tag a ensuite été révélé par la protéine fixatrice du S-tag couplé à l'alkaline phosphatase et par réaction colorimétrique. La protéine S-tag-PS2Nt se fixe bien sur les peptides Aβ dans ce test in vitro (Fig8A). Comme contrôles, des duplicats ont été incubés en présence d'un extrait bactérien n'exprimant que le peptide S qui est utilisé comme niveau de fixation non-spécifique sur le peptide Aβ (formes 1-40 et 1-42). Des dilutions sérielles de l'extrait bactérien permettent d'établir que cette fixation est dose-dépendante et saturable. Dans cette expérience, la fixation semble être plus importante sur le Aβ₁₋₄₂ que sur le Aβ₁₋₄₀ avec cependant une certaine variabilité. Par exemple, la fixation de PS2Nt est dépendante de la dose de Aβ déposée sur la membrane, alors que Aβ₁₋₄o et Aβ₁₋₄₂ présentent des valeurs équivalentes de fixation.

Cet exemple fourni donc une démonstration de la reconstitution de l'interaction PS2Nt-APP(Aβ) in vitro entre Aβ synthétique et la PS2NT d'origine bactérienne. Etant donné que les mutations pathologiques de PS2 conduisent à une augmentation du ratio Aβ1-42/Aβ1-40 produit dans de nombreux systèmes et que par ailleurs, il y a interaction physique entre PS2 et APP, il apparaît que cette interaction physique pourrait être impliquée dans la production du peptide Aβ₁₋₄₂. Ainsi, l'inhibition de cette interaction constitue une approche thérapeutique extrémement originale pour la maladie d'Alzheimer.

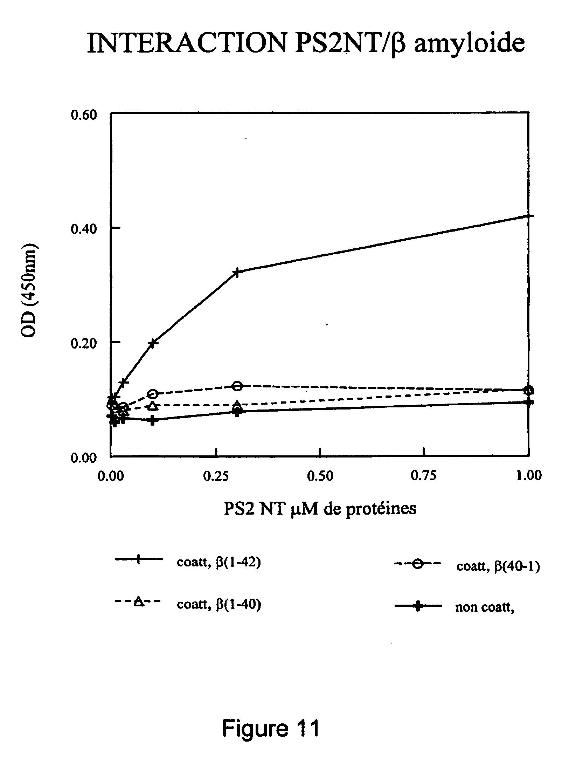

### Exemple 6. Test d'interaction Aβ42/PS2NT en format 96 puits (type ELISA). Fig11

L'exemple 5, fournit des résultats démontrant l'interaction directe entre le peptide Aβ et la protéine PS2NT sur membrane de nitrocellulose. Cet exemple a pour but de confirmer les informations de l'exemple 5 et de décrire la mise en évidence de l'interaction dans un format de plaques 96 puits. Le peptide Aβ (Aβ40 ou Aβ42) est fixé par incubation sur plaques 96 puits plastique. Les plaques sont ensuite incubées avec la protéine PS2NT recombinante. Après rinçages, la détection de l'interaction (Aβ/PS2NT) se fait par fixation de la protéine fixatrice du S-tag dans les puits et révélation colorimétrique. PS2NT se fixe de façon dose-dépendante sur le peptide Aβ1-42 (Fig 11) mais pas sur le peptide Aβ1-40 ou sur le peptide de séquence inversée Aβ40-1, ni sur un autre peptide amyloidogène, l'amyline. Les quantités de peptides Aβ1-42 ou Aβ1-40 fixées sur les plaques sont identiques comme vérifié par immunodétection). La constante de fixation de PS2NT sur Aβ42 est de 0.18 µM. La spécificité de PS2NT pour la forme Aβ42 du peptide par rapport à Aβ40 n'avait été que suggérée dans l'exemple 5. Cet exemple établit cette spécificité qui est parfaitement reproductible dans le présent test.

Ce format de test d'interaction Aβ42/PS2NT permet donc d'envisager facilement un test de criblage de molécules qui inhibent cette interaction.

### Exemple 7. Test d'interaction A β42/PS2NT en format HTRF

Dans les exemples 5 et 6, l'interaction directe entre le peptide Aβ42 et la protéine PS2NT a été démontrée d'une part sur membrane de nitrocellulose et d'autre part sur plaques 96 puits (type ELISA).

Cet exemple a pour but de confirmer ces informations et décrit la mise en évidence de l'interaction en phase liquide/homogène en utilisant la technique de transfert de fluorescence.

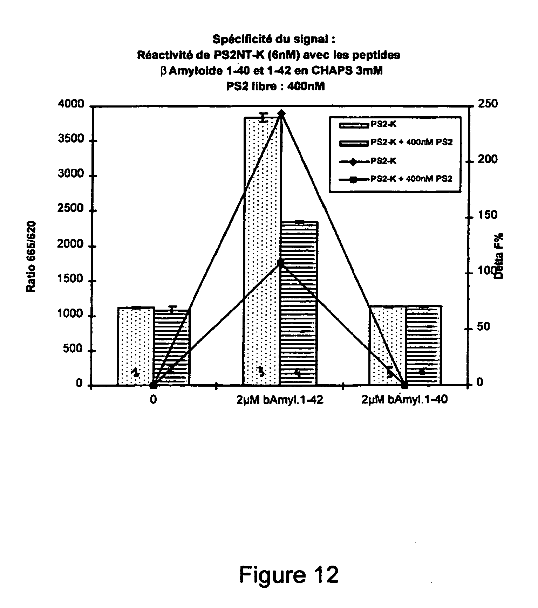

Le principe du test décrit dans Materiels et Méthodes (5.4) repose sur le transfert de fluorescence. La protéine recombinante PS2NT marquée au cryptate d'Europium (PS2NT-K) interagit avec le peptide biot-Aβ42 comme le montre la figure 12 (barre N°3). Ce signal est diminué et donc l'interaction PS2NT-K/biot-Aβ42 est déplacée par un excés de PS2NT non marquée (CI50=400nM ). Le signal de fluorescence détecté est stable dans le temps : de 4h à température ambiante à 24 h à 4°C (selon les conditions choisies, décrites dans Matériesl et Methodes ).

Cette interaction est dose-dépendante pour le peptide biot-Aβ42 (zone de linéarité du signal de 0 à 2.5µM) et pour PS2NT-K (zone de linéarité du signal de 0 à 7.5 nM). Comme l'indique la figure 12, barre N° 5, il n'y a pas d'interaction de PS2NT-K avec le peptide biot-Aβ40 apportant un élément supplémentaire de spécificité. La spécificité de PS2NT pour la forme Aβ42 du peptide par rapport à Aβ40 qui n'avait été que suggérée dans l'exemple 5 est donc confirmée dans l'exemple 6 et le présent exemple.

Ce test HTRF d'interaction entre PS2NT et Aβ42 (reflétant l'interaction APP/PS en cellules) permet donc l'identification de molécules chimiques inhibant cette interaction par un criblage à haut flux.

### Exemple 8. Reconstitution de l'interaction APP/PS1 in vitro

Cet exemple a pour but de démonter que l'interaction entre les protéines complètes APP et PS1 peut être recréée à partir de lysats cellulaires différents, mélangés uniquement pour mettre en évidence l'interaction.

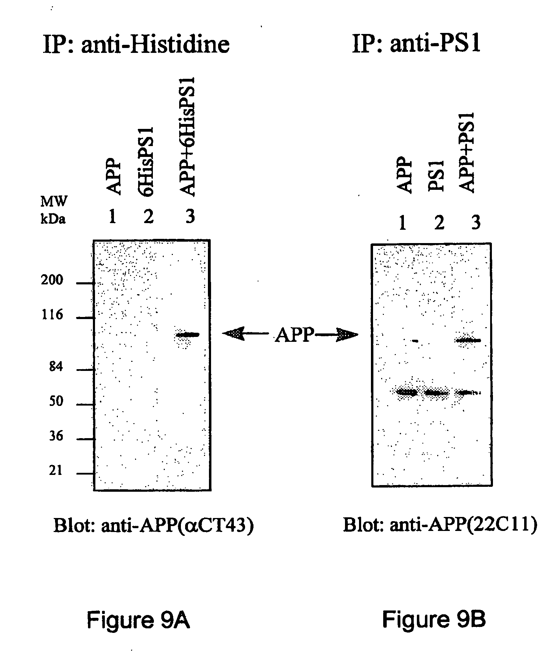

Le système d'expression baculovirus permettant l'expression de grandes quantités de protéines recombinantes, les lysats de cellules infectées individuellement par chacun des trois virus ont été utilisés comme source de protéines APP, PS1 et PS1-His6. Les fractions solubilisées contenant l'APP, PS1 ou 6HisPS1 sont mélangées, puis immunoprécipitées en présence d'anticorps anti Histidines ou antiPS1 pendant une nuit à 4°C. L'APP est clairement détecté dans les immunoprécipitats (Fig 9A, piste3 et Fig9B, piste 3) démontrant que l'interaction APP avec PS1-His ou PS1 est reconstituée in vitro par incubation des deux protéines. L'APP seul semble être faiblement précipité par l'anticorps anti-PS1 (Fig9B, piste 1) mais pas avec l'anticorps anti-His confirmant la spécificité de l'interaction dans ce cas-là. Ces résultats permettent la mise au point d'un test d'interaction in-vitro des deux protéines complétes APP et PS1.

### Exemple 9. SecPS2Nt bloque l'interaction de APP et PS1 en cellules transfectées.

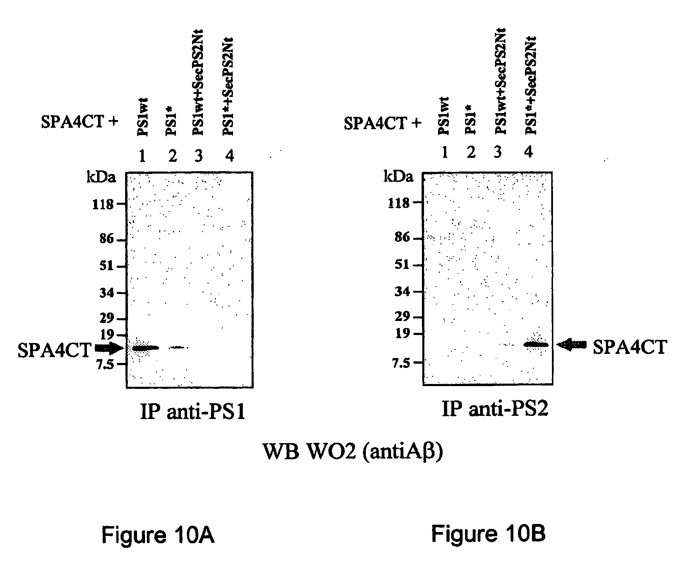

Il a été démontré dans les exemples précédents que APP interagit avec PS1 de façon semblable à PS2 et que pour cette dernière, la construction SecPS2Nt suffit à l'interaction avec APP. Cet exemple a pour but d'évaluer si la fixation de SecPS2Nt sur l'APP peut bloquer l'interaction avec PS1 de façon croisée (hétérologue). Dans le sytème COS1, le SPA4CT (correspondant aux 100 derniers résidus de l'APP précédés d'un peptide signal) peut être détecté dans les immunoprécipitats anti-PS1 des cellules exprimant SPA4CT et PS1 wt ou PS1 mutant, PS1* ,(Fig 10A. pistes 1 et 2). En outre, lorsque SecPS2NT est également cotransfecté, le signal SPA4CT disparait quasiment dans les immunoprécipitats anti-PS1 (Fig 10A, pistes 3 et 4). Après l'immunoprécipitation anti-PS1, les surnageants (fraction non-liée à la protéine A sépharose) ont subi une seconde immunoprécipitation avec l'anticorps anti-PS2. Le SPA4CT est clairement détecté dans les cellules cotransfectées avec PS1 et SecPS2Nt (Fig10B, pistes 3 et 4) démontrant que dans ces cellules, SecPS2Nt en se fixant, a déplacé la fixation de SPA4CT sur PS1. Cette expérience nous permet donc de conclure que SecPS2Nt est une molécule capable non seulement de se fixer sur APP, mais aussi de déplacer la fixation de APP sur PS1 et vraisemblablement PS2. SecPS2Nt peut donc en cellules servir de leurre pour bloquer l'interaction APP avec les deux présénilines, PS1 et PS2. En effet, les résultats de la cartographie de l'interaction PS1/APP, confirment que les zones d'interaction mises en jeu sont similaires à celles de PS2.

### Exemple 10. Le blocage de l'interaction APP/PS1 conduit à l'inhibition de la production du peptide amvloide Aβ42 intracellulaire.

L'exemple précédent démontre que l'expression de SecPS2NT peut bloquer l'interaction entre APP et PS1 ou PS1 mutant. Le présent exemple analyse les conséquences de cette inhibition sur la production du peptide amyloïde, en particulier de ces deux formes Aβ40 et Aβ42. En effet, il a été précédemment décrit dans la littérature que les mutations pathologiques des présénilines (PS1 ou PS2) conduisaient à une augmentation du ratiode la forme longue du peptide Aβ, forme Aβ42, sur la forme Aβ40. ratio Aβ42/Aβ40 (Borchelt et coll. 1996 et pour revue Hardy, 1997).

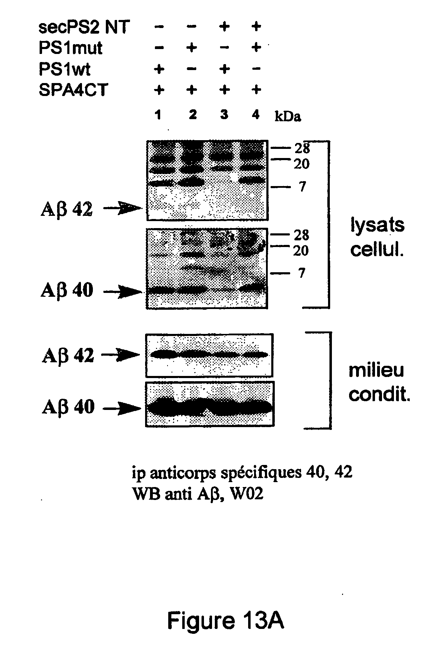

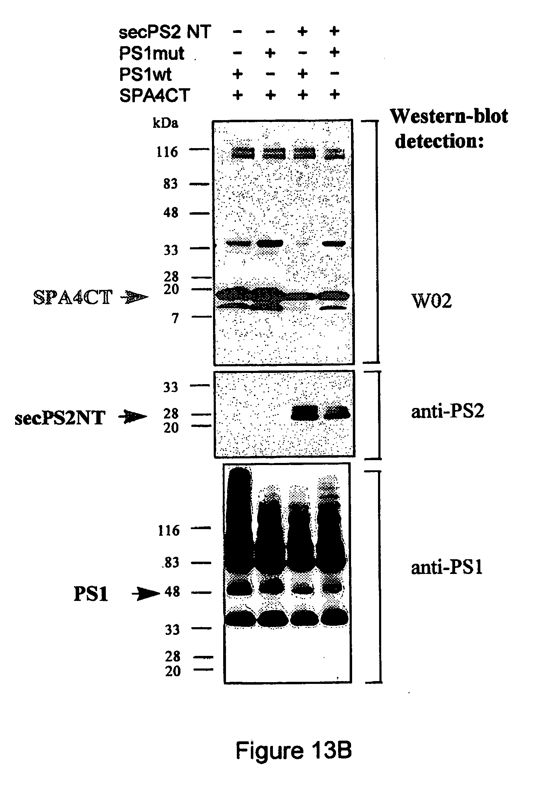

SPA4CT a été coexprimé avec PS1 wt (Fig 13A, pistes 1 & 3) ou avec PS1 mutant (Fig 13A, pistes 2 & 4) soit en l'absence (pistes 1 & 2) soit en présence de SecPS2NT (pistes 3 & 4). Les lysats cellulaires et les milieux conditionnés des cellules ont été analysés pour la production du peptide amyloïde. Les formes Aβ40 et Aβ42 ont été analysée par immunoprécipitation avec des anticorps reconnaissant spécifiquement les extrémités C-terminales Aβ40 (FCA3340) ou Aβ42 (FCA3542, Barelli et coll., 1997) et les immunoprécipitats analysés par immunoblot avec un anticorps reconnaissant les deux formes. Dans les lysats cellulaires, l'expression de PS1 mutant conduit bien à une augmentation de la production de Aβ42 (1.5 à 2 fois) et de ses formes multimériques par rapport à PS1wt et peu de variation des niveaux de Aβ40 (comparer Fig 13 A, pistes 1 et 2, panels Aβ42 et Aβ40 lysats cellulaires) comme attendu. En présence de SecPS2NT , les niveaux de Aβ42 (et multimères) sont considérablement réduits (Fig 13A, pistes 3 et 4) autant avec PS1 wt que PS1 mutant. Dans le milieu extracellulaire, les niveaux de Aβ42 semblent aussi diminués mais de façon moins importante. Il n'y a pas de variation des niveaux de peptide amyloïde Aβ40 entre les différentes conditions ce qui démontre que l'effet sur le Aβ42 est spécifique et n'est pas dû à une modification globale des niveaux d'expression. Ceci est confirmé par l'analyse de l'expression des différents gènes transfectés: SPA4CT, SecPS2NT et PS1 (Fig 13B). Dans cet exemple, il a donc été démontré qu'inhiber l'interaction PS1/APP avec le dominant génétique SecPS2NT conduit à une diminution des niveaux de production de Aβ42 intracellulaire autant avec PS 1 mutant qu'avec PS1 wt. En regard du rôle primordial accordé au Aβ42 dans le développement de la maladie d'Alzheimer, cet exemple apporte la démonstration que l'inhibition de l'interaction APP/PS représente donc une cible thérapeutique importante autant pour les formes génétiques que les formes sporadiques de la maladie.

### Exemple 11. Détection de l'interaction de PS2 avec l'APP endogène des cellules COS à l'aide d'un traitement pharmacologique.

Cet exemple a pour but de démontrer que les résulats obtenus dans les exemples précédents (ces résultats en cellules, correspondaient à la surexpression des deux partenaires de l'interaction APP et PS (PS1 ou PS2)) sont également valables avec des protéines non surexprimées ou endogènes. En effet, la forte surexpression des deux protéines pourrait conduire à un artefact d'interaction. La détection de l'interaction dans des conditions n'impliquant pas la surexpression simultanée des deux partenaires a été recherchée dans cet exemple.

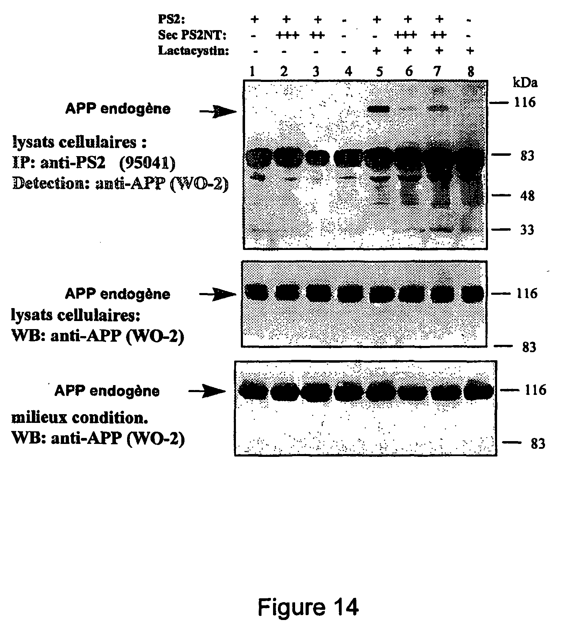

Les cellules COS expriment l'APP de façon endogène bien qu'à de faibles niveaux. Les cellules COS ont donc été tranfectées avec PS2 seulement. Les lysats cellulaires ont été analysés par immunoprécipitation avec l'anticorps dirigé contre le peptide du N-term de PS2 et révélés par immunoblotting avec l'anticorps anti-APP, WO2. Le panneau supérieur de la figure 14 montre que la transfection avec PS2 seul ne permet pas de détecter d'interaction avec APP endogène par coimmunoprécipitation (Fig 14, piste 1).

Par ailleurs, puisque l'interaction APP/PS conduit à la production du peptide amyloide Aβ42 (exemple précédent), donc au catabolisme de l'APP, au niveau du reticulum endoplasmique, le protéasome qui est le système de dégradation protéolytique dans ce compartiment cellulaire pourrait être impliqué. L'effet de la lactacystine, un inhibiteur séléctif du protéasome a été analysé. Après incubation des cellules transfectées avec PS2 en présence de lactacystine, l'APP endogène des cellules COS peut être clairement mis en évidence dans les immunoprécipitats PS2 (Fig 14, piste 5, bande à 110 kDa). Cette interaction avec l'APP endogène présente les mêmes caractéristiques que précédemment puisqu'elle peut-être déplacée par le dominant génétique SecPS2NT (Fig 14, pistes 6 et 7) avec un effet dose-dépendant. En effet, la piste 7 montre qu'à un dosage modéré de SecPS2NT, une bande de faible intensité correspondant à l'APP endogène est toujours visible. A un dosage plus important de SecPS2NT (piste 6), la bande correspondant à l'APP endogène apparait très faiblement et démontre le caractère dosage dépendant. Un faible signal résiduel APP est toujours présent (bande à environ 110 kDa) et est dû au complexe entre APP et SecPS2NT qui est rapidement sécrété (puisque SecPS2NT n'a plus de domaines transmembranaires d'ancrage) et donc ne s'accumule pas intracellulairement.

La Figure 14 (paneaux milieu et inférieur) montre que le traitement à la lactacystine n'affecte pas les niveaux d'APP totaux autant cellulaires que sécrétés. En effet, les bandes correspondant aux niveaux d'expression de l'APP sont quasiment constantes en intensité. La spécificité de l'effet lactacystine est ainsi démontré sur la sous-population d'APP en interaction avec PS2.

Par ces résultats, il a pu être démontré que l'interaction APP/PS2 peut être détectée avec l'APP endogène des cellules COS si le protéasome est inhibé. En outre, ces résultats démontrent que l'interaction APP/PS2 peut être détectée dans des conditions moins artificielles. Cependant cette interaction est trés labile. Aussi, afin d'obtenir une détection plus marquée, il a été recouru soit à un inhibiteur de dégradation protéolytique dans le présent exemple soit à la surexpression des deux partenaires dans les exemples précédents. Cet exemple démontre donc que les résulats obtenus dans les exemples précédents avec surexpression en cellule des deux partenaires de l'interaction (APP et PS1 ou PS2) sont également valables avec des protéines non surexprimées ou endogènes.

### Exemple 12. PS2 interagit avec un deuxième segment de APP, autre que Aβ.

Cet exemple a pour but de démonter qu'un autre segment de APP interagit avec PS2.

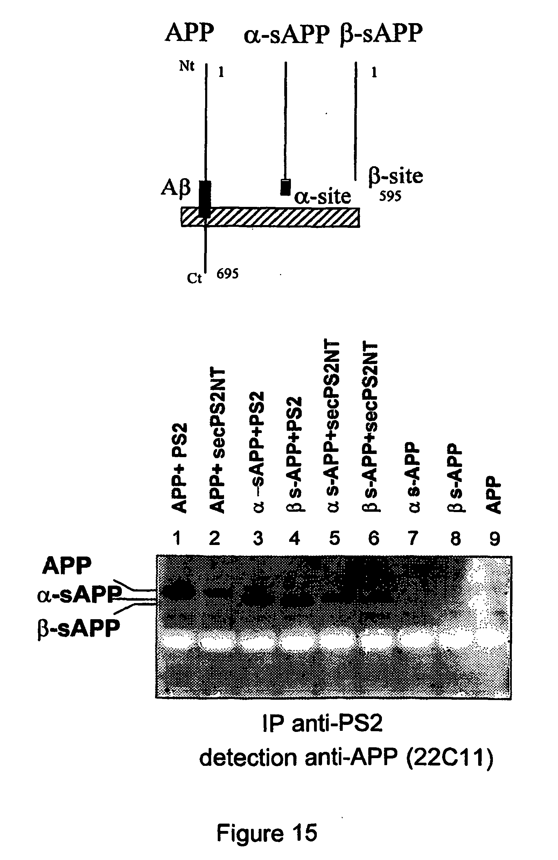

Il a été précédemment démontré que SecPS2NT interagit dans le milieu extracellulaire avec les formes sécrétées de APP (Fig3A, piste 4). Les formes sécétées de l'APP sont libérées après clivage soit au site β (position 595), correspondant au début du peptide Aβ, soit au site α (position 612) au sein même de ce peptide. Ces résultats suggèrent que PS2NT interagit aussi avec une région N-term de l'APP différente de Aβ. Des formes tronquées de APP ont été construites, par insertion d'un codon stop aux sites β (β-sAPP) et α (α-sAPP) et ont été testées. PS2 complet et SecPS2NT interagissent effectivement avec α-sAPP (Fig 15, pistes 3 et 5) et β-sAPP (Fig 15, pistes 4 et 6). Ces résultats établissent qu'un segment de APP compris entre la position 1 et 595 (et donc autre que le Aβ) est également capable d'interagir avec PS2 et PS2NT. Ces résultats permettent en outre de confirmer que l'interaction PS2NT/APP peut avoir lieu en absence d'ancrage à la membrane des deux partenaires et dans le compartiment luminal (ou extracellulaire) de la cellule.

### Références

- Doan et col. (1996) Protein topology of presenilinl. Neuron 17:1023-1030.
- Thinakaran et col., (1996) Endoproteolysis of Presenilin 1 and accumulation of processed derivatives in vivo. Neuron 17:181-190.
- Podlisny et col., (1997) Presenilin proteins undergo heterogeneous endoproteolysis between Thr291 and Ala299 and occur as stable N- and C-terminal fragments in normal and Alzheimer brain tissue. Neurobiology of Disease 3:325-337.
- Pradier, L., Czech, C., Mercken, L., Revah, F. and Imperato, A. (1996) Biochemical characterization of presenilins (S182 and STM2) proteins. Neurobiol. Aging 17: S 137
- Scheuner et coll. (1996) Secreted amyloid b-protein similar to that in the senile plaques of Alzheimer's disease is increased in vivo by the presenilin 1 and 2 and APP mutations linked to familial Alzheimer's disease. Nature Med. 2: 864-870.
- Dyrks, T., Dyrks, E., Mönning, U., Urmoneit, B., Turner, J. and Beyreuther, K. (1993) Generation of bA4 from the amyloid protein precursor and fragment thereof FEBS Lett. 335:89-93.
- Weidemann, A., Paliga, K., Dürrwang, U., Czech, C., Evin, G., Masters, C., & Beyreuther, K. (1997). Formation of stable complexes between two Alzheimer's disease gene products: Presenilin-2 and b-Amyloid precursor protein. Nature Medicine 3: 328-332
- Blanchard, V., Czech, C., Bonici, B., Clavel, N., Gohin, M., Dalet, K., Revah, F., Pradier, L., Imperato, A. and S. Moussaoui. (1997) Immunohistochemical analysis of presenilin 2 expression in the mouse brain: distribution pattern and co-localization with presenilin 1 protein. Brain Res. 758:209-217.
- Borchelt, D.R., Thinakaran, G., Eckman, C., Lee, M.K., Davenport, F., Ratovitsky, T., Prada, C.-M., Kim, G., Seekins, S., Yager, D., Slunt, H.H., Wang, R., Seeger, M., Levey, A.I., Gandy, S.E., Copeland, N.G., Jenkins, N., Price, D.L., Younkin, S.G. and S. Sisodia (1996) Familial Alzheimer's diseaselinked presenilin 1 variants elevate Ab1-42/1-40 ratio in vitro and in vivo. Neuron 17: 1005-1013
- Duff, K., Eckman, C., Zehr, C., Yu, X., Prada, C.-M., Perez-tur, J., Hutton, M., Buee, L; Harigaya, Y., Yager, D., Morgan, D., Gordon, M.N., Holcomb, L., Refolo, L., Zenk, B., Hardy, J. and S. Younkin (1996) Increased amyloid-b42(43) in brains of mice expressing mutant presenilin 1. Nature 383:710-713.
- Hardy, J. (1997) Amyloid, the presenilins and Alzheimer's disease. Trends in Neurosci. 20: 154-159.
- Stephens, D.J. and B.M. Austen (1996) Metabolites of the b-amyloid precursor protein generated by b-secretase localise to the Trans-Golgi Network and late endosome in 293 cells. J. Neurosci. Res 46:211-225.
- Essalmani, R., Guillaume, J.-M., Mercken, L., and Octave, J.-N. (1996). Baculovirus-Infected Cells Do not Produce the Amyloid Peptide of Alzheimer's Disease from its Precursor. FEBS Lett. 389: 157-161.
- Barelli et coll., (1997), Characterization of new polyclonal antibodies specific for 40 and 42 amino acid-long amyloid β peptides : their use to examine the cell biology of presenilins and the immunohistochemistry of sporadic Alzheimer's disease and cerebral amyloid angiopathy cases. Mo/ecu/ar Medecine 3:695-707.

### LISTE DE SEQUENCES

(1) INFORMATIONS GENERALES:
   (i) DEPOSANT:
      (A) NOM: RHONE-POULENC RORER
      (B) RUE: 20 Avenue Raymond Aron
      (C) VILLE: Antony
      (E) PAYS: France
      (F) CODE POSTAL: 92165
      (H) TELECOPIE: 01.55.71.72.91
   (ii) TITRE DE L' INVENTION: Peptides capables d'inhiber l'interaction entre les presenilines et le precurseur du peptide beta-amyloide et ou le peptide beta-amyloide et test d'interactionpour la recherche de molécules inhibitricesde ladite interaction.
   (iii) NOMBRE DE SEQUENCES: 11
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 261 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (iv) ANTI-SENS: NON
   (ix) CARACTERISTIQUE:
      (A) NOM/CLE: CDS
      (B) EMPLACEMENT:1..261
   (xi) DESCRIPTION DE LA SEQUENCE: SEQ ID NO: 1:
   ATG CTC ACA TTC ATG GCC TCT GAC AGC GAG GAA GAA GTG TGT GAT GAG      48
   Met Leu Thr Phe Met Ala Ser Asp Ser Glu Glu Glu Val Cys Asp Glu
     1               5                  10                  15
   CGG ACG TCC CTA ATG TCG GCC GAG AGC CCC ACG CCG CGC TCC TGC CAG      96
   Arg Thr Ser Leu Met Ser Ala Glu Ser Pro Thr Pro Arg Ser Cys Gln
                20                  25                  30
   GAG GGC AGG CAG GGC CCA GAG GAT GGA GAG AAT ACT GCC CAG TGG AGA     144
   Glu Gly Arg Gln Gly Pro Glu Asp Gly Glu Asn Thr Ala Gln Trp Arg
            35                  40                  45
   AGC CAG GAG AAC GAG GAG GAC GGT GAG GAG GAC CCT GAC CGC TAT GTC     192
   Ser Gln Glu Asn Glu Glu Asp Gly Glu Glu Asp Pro Asp Arg Tyr Val
        50                  55                  60
   TGT AGT GGG GTT CCC GGG CGG CCG CCA GGC CTG GAG GAA GAG CTG ACC     240
   Cys Ser Gly Val Pro Gly Arg Pro Pro Gly Leu Glu Glu Glu Leu Thr
    65                  70                  75                  80
   CTC AAA TAC GGA GCG AAG CAT                                         261
   Leu Lys Tyr Gly Ala Lys His
                    85
(2) INFORMATIONS POUR LA SEQ ID NO: 2:
   (i) CARACTERISTIQUES DE LA SEQUENCE:
      (A) LONGUEUR: 243 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (iv) ANTI-SENS: NON
   (ix) CARACTERISTIQUE:
      (A) NOM/CLE: CDS
      (B) EMPLACEMENT:1..243
   (xi) DESCRIPTION DE LA SEQUENCE: SEQ ID NO: 2:
   ATG ACA GAG TTA CCT GCA CCG TTG TCC TAC TTC CAG AAT GCA CAG ATG     48
   Met Thr Glu Leu Pro Ala Pro Leu Ser Tyr Phe Gln Asn Ala Gln Met
     1               5                  10                  15
   TCT GAG GAC AAC CAC CTG AGC AAT ACT GTA CGT AGC CAG AAT GAC AAT     96
   Ser Glu Asp Asn His Leu Ser Asn Thr Val Arg Ser Gln Asn Asp Asn
                20                  25                  30
   AGA GAA CGG CAG GAG CAC AAC GAC AGA CGG AGC CTT GGC CAC CCT GAG    144
   Arg Glu Arg Gln Glu His Asn Asp Arg Arg Ser Leu Gly His Pro Glu
            35                  40                  45
   CCA TTA TCT AAT GGA CGA CCC CAG GGT AAC TCC CGG CAG GTG GTG GAG    192
   Pro Leu Ser Asn Gly Arg Pro Gln Gly Asn Ser Arg Gln Val Val Glu
        50                  55                  60
   CAA GAT GAG GAA GAA GAT GAG GAG CTG ACA TTG AAA TAT GGC GCC AAG    240
   Gln Asp Glu Glu Glu Asp Glu Glu Leu Thr Leu Lys Tyr Gly Ala Lys
    65                  70                  75                  80
   CAT                                                                   243
   His
(2) INFORMATIONS POUR LA SEQ ID NO: 3:
   (i) CARACTERISTIQUES DE LA SEQUENCE:
      (A) LONGUEUR: 2088 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (iv) ANTI-SENS: NON
   (ix) CARACTERISTIQUE:
      (A) NOM/CLE: CDS
      (B) EMPLACEMENT:1..2088
   (xi) DESCRIPTION DE LA SEQUENCE: SEQ ID NO: 3:
   ATG CTG CCC GGT TTG GCA CTG CTC CTG CTG GCC GCC TGG ACG GCT CGG    48
   Met Leu Pro Gly Leu Ala Leu Leu Leu Leu Ala Ala Trp Thr Ala Arg
    1               5                  10                  15
   GCG CTG GAG GTA CCC ACT GAT GGT AAT GCT GGC CTG CTG GCT GAA CCC    96
   Ala Leu Glu Val Pro Thr Asp Gly Asn Ala Gly Leu Leu Ala Glu Pro
               20                  25                  30
   CAG ATT GCC ATG TTC TGT GGC AGA CTG AAC ATG CAC ATG AAT GTC CAG   144
   Gln Ile Ala Met Phe Cys Gly Arg Leu Asn Met His Met Asn Val Gln
           35                  40                  45
   AAT GGG AAG TGG GAT TCA GAT CCA TCA GGG ACC AAA ACC TGC ATT GAT   192
   Asn Gly Lys Trp Asp Ser Asp Pro Ser Gly Thr Lys Thr Cys Ile Asp
       50                  55                  60
   ACC AAG GAA GGC ATC CTG CAG TAT TGC CAA GAA GTC TAC CCT GAA CTG   240
   Thr Lys Glu Gly Ile Leu Gln Tyr Cys Gln Glu Val Tyr Pro Glu Leu
   65                  70                  75                  80
   CAG ATC ACC AAT GTG GTA GAA GCC AAC CAA CCA GTG ACC ATC CAG AAC   288
   Gln Ile Thr Asn Val Val Glu Ala Asn Gln Pro Val Thr Ile Gln Asn
                   85                  90                  95
   TGG TGC AAG CGG GGC CGC AAG CAG TGC AAG ACC CAT CCC CAC TTT GTG   336
   Trp Cys Lys Arg Gly Arg Lys Gln Cys Lys Thr His Pro His Phe Val
               100                 105                 110
   ATT CCC TAC CGC TGC TTA GTT GGT GAG TTT GTA AGT GAT GCC CTT CTC   384
   Ile Pro Tyr Arg Cys Leu Val Gly Glu Phe Val Ser Asp Ala Leu Leu
           115                 120                 125
   GTT CCT GAC AAG TGC AAA TTC TTA CAC CAG GAG AGG ATG GAT GTT TGC   432
   Val Pro Asp Lys Cys Lys Phe Leu His Gln Glu Arg Met Asp Val Cys
       130                 135                 140
   GAA ACT CAT CTT CAC TGG CAC ACC GTC GCC AAA GAG ACA TGC AGT GAG   480
   Glu Thr His Leu His Trp His Thr Val Ala Lys Glu Thr Cys Ser Glu
   145                 150                 155                 160
   AAG AGT ACC AAC TTG CAT GAC TAC GGC ATG TTG CTG CCC TGC GGA ATT   528
   Lys Ser Thr Asn Leu His Asp Tyr Gly Met Leu Leu Pro Cys Gly Ile
                   165                 170                 175
   GAC AAG TTC CGA GGG GTA GAG TTT GTG TGT TGC CCA CTG GCT GAA GAA   576
   Asp Lys Phe Arg Gly Val Glu Phe Val Cys Cys Pro Leu Ala Glu Glu
               180                 185                 190
   AGT GAC AAT GTG GAT TCT GCT GAT GCG GAG GAG GAT GAC TCG GAT GTC   624
   Ser Asp Asn Val Asp Ser Ala Asp Ala Glu Glu Asp Asp Ser Asp Val
           195                 200                 205
   TGG TGG GGC GGA GCA GAC ACA GAC TAT GCA GAT GGG AGT GAA GAC AAA   672
   Trp Trp Gly Gly Ala Asp Thr Asp Tyr Ala Asp Gly Ser Glu Asp Lys
       210                 215                 220
   GTA GTA GAA GTA GCA GAG GAG GAA GAA GTG GCT GAG GTG GAA GAA GAA    720
   Val Val Glu Val Ala Glu Glu Glu Glu Val Ala Glu Val Glu Glu Glu
   225                 230                 235                 240
   GAA GCC GAT GAT GAC GAG GAC GAT GAG GAT GGT GAT GAG GTA GAG GAA    768
   Glu Ala Asp Asp Asp Glu Asp Asp Glu Asp Gly Asp Glu Val Glu Glu
                   245                 250                 255
   GAG GCT GAG GAA CCC TAC GAA GAA GCC ACA GAG AGA ACC ACC AGC ATT    816
   Glu Ala Glu Glu Pro Tyr Glu Glu Ala Thr Glu Arg Thr Thr Ser Ile
               260                 265                 270
   GCC ACC ACC ACC ACC ACC ACC ACA GAG TCT GTG GAA GAG GTG GTT CGA    864
   Ala Thr Thr Thr Thr Thr Thr Thr Glu Ser Val Glu Glu Val Val Arg
           275                 280                 285
   GTT CCT ACA ACA GCA GCC AGT ACC CCT GAT GCC GTT GAC AAG TAT CTC    912
   Val Pro Thr Thr Ala Ala Ser Thr Pro Asp Ala Val Asp Lys Tyr Leu
       290                 295                 300
   GAG ACA CCT GGG GAT GAG AAT GAA CAT GCC CAT TTC CAG AAA GCC AAA    960
   Glu Thr Pro Gly Asp Glu Asn Glu His Ala His Phe Gln Lys Ala Lys
   305                 310                 315                 320
   GAG AGG CTT GAG GCC AAG CAC CGA GAG AGA ATG TCC CAG GTC ATG AGA   1008
   Glu Arg Leu Glu Ala Lys His Arg Glu Arg Met Ser Gln Val Met Arg
                   325                 330                 335
   GAA TGG GAA GAG GCA GAA CGT CAA GCA AAG AAC TTG CCT AAA GCT GAT   1056
   Glu Trp Glu Glu Ala Glu Arg Gln Ala Lys Asn Leu Pro Lys Ala Asp
               340                 345                 350
   AAG AAG GCA GTT ATC CAG CAT TTC CAG GAG AAA GTG GAA TCT TTG GAA   1104
   Lys Lys Ala Val Ile Gln His Phe Gln Glu Lys Val Glu Ser Leu Glu
           355                 360                 365
   CAG GAA GCA GCC AAC GAG AGA CAG CAG CTG GTG GAG ACA CAC ATG GCC   1152
   Gln Glu Ala Ala Asn Glu Arg Gln Gln Leu Val Glu Thr His Met Ala
       370                 375                 380
   AGA GTG GAA GCC ATG CTC AAT GAC CGC CGC CGC CTG GCC CTG GAG AAC   1200
   Arg Val Glu Ala Met Leu Asn Asp Arg Arg Arg Leu Ala Leu Glu Asn
   385                 390                 395                 400
   TAC ATC ACC GCT CTG CAG GCT GTT CCT CCT CGG CCT CGT CAC GTG TTC   1248
   Tyr Ile Thr Ala Leu Gln Ala Val Pro Pro Arg Pro Arg His Val Phe
                   405                 410                 415
   AAT ATG CTA AAG AAG TAT GTC CGC GCA GAA CAG AAG GAC AGA CAG CAC   1296
   Asn Met Leu Lys Lys Tyr Val Arg Ala Glu Gln Lys Asp Arg Gln His
               420                 425                 430
   ACC CTA AAG CAT TTC GAG CAT GTG CGC ATG GTG GAT CCC AAG AAA GCC   1344
   Thr Leu Lys His Phe Glu His Val Arg Met Val Asp Pro Lys Lys Ala
           435                 440                 445
   GCT CAG ATC CGG TCC CAG GTT ATG ACA CAC CTC CGT GTG ATT TAT GAG   1392
   Ala Gln Ile Arg Ser Gln Val Met Thr His Leu Arg Val Ile Tyr Glu
       450                 455                 460
   CGC ATG AAT CAG TCT CTC TCC CTG CTC TAC AAC GTG CCT GCA GTG GCC   1440
   Arg Met Asn Gln Ser Leu Ser Leu Leu Tyr Asn Val Pro Ala Val Ala
   465                 470                 475                 480
   GAG GAG ATT CAG GAT GAA GTT GAT GAG CTG CTT CAG AAA GAG CAA AAC   1488
   Glu Glu Ile Gln Asp Glu Val Asp Glu Leu Leu Gln Lys Glu Gln Asn
                   485                 490                 495
   TAT TCA GAT GAC GTC TTG GCC AAC ATG ATT AGT GAA CCA AGG ATC AGT   1536
   Tyr Ser Asp Asp Val Leu Ala Asn Met Ile Ser Glu Pro Arg Ile Ser
               500                 505                 510
   TAC GGA AAC GAT GCT CTC ATG CCA TCT TTG ACC GAA ACG AAA ACC ACC   1584
   Tyr Gly Asn Asp Ala Leu Met Pro Ser Leu Thr Glu Thr Lys Thr Thr
           515                 520                 525
   GTG GAG CTC CTT CCC GTG AAT GGA GAG TTC AGC CTG GAC GAT CTC CAG   1632
   Val Glu Leu Leu Pro Val Asn Gly Glu Phe Ser Leu Asp Asp Leu Gln
       530                 535                 540
   CCG TGG CAT TCT TTT GGG GCT GAC TCT GTG CCA GCC AAC ACA GAA AAC   1680
   Pro Trp His Ser Phe Gly Ala Asp Ser Val Pro Ala Asn Thr Glu Asn
   545                 550                 555                 560
   GAA GTT GAG CCT GTT GAT GCC CGC CCT GCT GCC GAC CGA GGA CTG ACC   1728
   Glu Val Glu Pro Val Asp Ala Arg Pro Ala Ala Asp Arg Gly Leu Thr
                   565                 570                 575
   ACT CGA CCA GGT TCT GGG TTG ACA AAT ATC AAG ACG GAG GAG ATC TCT   1776
   Thr Arg Pro Gly Ser Gly Leu Thr Asn Ile Lys Thr Glu Glu Ile Ser
               580                 585                 590
   GAA GTG AAG ATG GAT GCA GAA TTC CGA CAT GAC TCA GGA TAT GAA GTT   1824
   Glu Val Lys Met Asp Ala Glu Phe Arg His Asp Ser Gly Tyr Glu Val
           595                 600                 605
   CAT CAT CAA AAA TTG GTG TTC TTT GCA GAA GAT GTG GGT TCA AAC AAA   1872
   His His Gln Lys Leu Val Phe Phe Ala Glu Asp Val Gly Ser Asn Lys
       610                 615                 620
   GGT GCA ATC ATT GGA CTC ATG GTG GGC GGT GTT GTC ATA GCG ACA GTG   1920
   Gly Ala Ile Ile Gly Leu Met Val Gly Gly Val Val Ile Ala Thr Val
   625                 630                 635                 640
   ATC GTC ATC ACC TTG GTG ATG CTG AAG AAG AAA CAG TAC ACA TCC ATT   1968
   Ile Val Ile Thr Leu Val Met Leu Lys Lys Lys Gln Tyr Thr Ser Ile
                   645                 650                 655
   CAT CAT GGT GTG GTG GAG GTT GAC GCC GCT GTC ACC CCA GAG GAG CGC   2016
   His His Gly Val Val Glu Val Asp Ala Ala Val Thr Pro Glu Glu Arg
               660                 665                 670
   CAC CTG TCC AAG ATG CAG CAG AAC GGC TAC GAA AAT CCA ACC TAC AAG   2064
   His Leu Ser Lys Met Gln Gln Asn Gly Tyr Glu Asn Pro Thr Tyr Lys
           675                 680                 685
   TTC TTT GAG CAG ATG CAG AAC TAG                                   2088
   Phe Phe Glu Gln Met Gln Asn  *
       690                 695
(2) INFORMATIONS POUR LA SEQ ID NO: 4: oligo
   (i) CARACTERISTIQUES DE LA SEQUENCE:
      (A) LONGUEUR: 38 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..38
   (xi) DESCRIPTION DE LA SEQUENCE: SEQ ID NO: 4:
      CGGAATTCATCGATTCCACCATGCTCACATTCATGGCC 38
(2) INFORMATIONS POUR LA SEQ ID NO: 5: oligo
   (i) CARACTERISTIQUES DE LA SEQUENCE:
      (A) LONGUEUR: 43 paires de bases
      (B) TYPE: nucléotide
      (C) NOMBRE DE BRINS: simple
      (D) CONFIGURATION: linaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..43
   (xi) DESCRIPTION DE LA SEQUENCE: SEQ ID NO: 5:
      CCGCTCGAGTCATTGTCGACCATGCTTCGCTCCGTATTTGAGG 43
(2) INFORMATIONS POUR LA SEQ ID NO: 6: oligo 8172
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..27
   (xi) DESCRIPTION DE LA SEQUENCE: SEQ ID NO: 6:
      CAAAGATCTGATGCAGAATTCCGACAT 27
(2) INFORMATIONS POUR LA SEQ ID NO: 7: oligo 8181
   (i) CARACTERISTIQUES DE LA SEQUENCE:
      (A) LONGUEUR: 59 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..59
   (xi) DESCRIPTION DE LA SEQUENCE: SEQ ID NO: 7:
      CAAGCGGCCGCTCATCCCTTGTCATCGTCGTCCTTGTAGTCTCCGTTCTGCATCTGCTC 59
(2) INFORMATIONS POUR LA SEQ ID NO: 8: oligo 8171
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..27
   (xi) DESCRIPTION DE LA SEQUENCE: SEQ ID NO: 8:
      CAAAGATCTAAGAAACAGTACACATCC 27
(2) INFORMATIONS POUR LA SEQ ID NO: 9: oligo
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..29
   (xi) DESCRIPTION DE LA SEQUENCE: SEQ ID NO: 9:
      ccatcgatggctaCATCTTCACTTCAGAG 29
(2) INFORMATIONS POUR LA SEQ ID NO: 10: oligo
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..31
   (xi) DESCRIPTION DE LA SEQUENCE: SEQ ID NO: 10:
      ccatcgatggctaTTTTTGATGATGAACTTC 31
(2) INFORMATIONS POUR LA SEQ ID NO: 11:oligo
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT:1..17
   (xi) DESCRIPTION DE LA SEQUENCE: SEQ ID NO: 11:
      CCGTGGAGCTCCTCCCG 17

## Revendications

1. Procédé de mise en évidence ou d'isolement de composés destinés au traitement de la maladie d'Alzhaimer comprenant au moins une étape de détection de l'inhibition de l'interaction entre l'APP et un peptide comprenant la séquence d'acides aminés 1 à 213 de PS1 ou la sequence d'acides aminés 1 à 87 de PS2

2. Procédé de mise en évidence ou d'isolement de composés capables d'inhiber au moins en partie l'interaction entre une préséniline et le peptide β-amyloïde, **caractérisé en ce qu'**il comprend au moins une étape de marquage des présénilines ou des fragments de celles-ci et une étape de détection de l'inhibition de l'interaction entre le peptide Aβ₁₋₄₂ et l'extrémité N-temninale des présenilines

3. Procédé selon l'une quelconque des revendications 1 à 2 **caractérisé en ce que** la préséniline est PS2.

4. Procédé selon l'une quelconque des revendications 1 à 3 **caractérisé en ce que** l'extrémité N-terminale de la préséniline est la partie 1-87 de la PS2.

5. Procédé selon l'une quelconque des revendications 2 à 4 **caractérisé en ce qu'**il comprend les étapes suivantes :
- le peptide Aβ₁₋₄₂ est absorbé au préalable sur une membrane de nitrocellulose par incubation.
- un extrait bactérien contenant tout ou partie d'une préseniline est ajouté pour incubation avec la molécule ou un mélange contenant différentes molécules à tester
- l'interaction de la préseniline avec le peptide Aβ₁₋₄₂ sur le filtre de nitrocellulose est mise en évidence à l'aide de protéines marqueur des présénilines.

6. Procédé selon la revendication 5 **caractérisé en ce que** l'une des protéines marqueurs est la protéine fixatrice du S-tag, couplée à la phosphatase alkaline.

## Claims

1. Process for revealing or isolating compounds for treating Alzheimer's disease, comprising at least one step of detecting inhibition of the interaction between APP and a peptide comprising the sequence of amino acids 1 to 213 of PS1 or the sequence of amino acids 1 to 87 of PS2.

2. Process for revealing or isolating compounds capable of at least partly inhibiting the interaction between a presenilin and the ß-amyloid peptide, **characterized in that** it comprises at least one step of marking the presenilins or fragments thereof and a step of detecting inhibition of the interaction between the peptide Aß₁₋₄₂ and the N-terminal end of the presenilins.

3. Process according to either of Claims 1 and 2, **characterized in that** the presenilin is PS2.

4. Process according to any one of Claims 1 to 3, **characterized in that** the N-terminal end of the presenilin is part 1-87 of PS2.

5. Process according to any one of Claims 2 to 4, **characterized in that** it comprises the following steps:
- the peptide Aß₁₋₄₂ is first absorbed onto a nitrocellulose membrane by incubation,
- a bacterial extract containing all or part of a presenilin is added for incubation with the molecule or a mixture containing different molecules to be tested,
- the interaction of the presenilin with the peptide Aß₁₋₄₂ on the nitrocellulose filter is revealed using presenilin marker proteins.

6. Process according to Claim 5, **characterized in that** one of the marker proteins is the S-tag fixing protein, coupled to alkaline phosphatase.

## Patentansprüche

1. Verfahren zum Nachweis oder zur Isolation von Verbindungen für die Behandlung von Alzheimer-Krankheit, bei dem man in mindestens einem Schritt die Hemmung der Wechselwirkung zwischen APP und einem Peptid nachweist, das die Aminosäuresequenz 1 bis 213 von PS1 oder die Aminosäuresequenz 1 bis 87 von PS2 umfasst.

2. Verfahren zum Nachweis oder zur Isolation von Verbindungen, die die Wechselwirkung zwischen einem Präsenilin und dem β-Amyloid-Peptid mindestens teilweise hemmen können, **dadurch gekennzeichnet, dass** es mindestens einen Schritt umfasst, in dem die Präseniline oder deren Fragmente markiert werden, und einen Schritt, in dem man die Hemmung der Wechselwirkung zwischen dem Peptid Aß₁₋₄₂ und dem N-Terminus der Präseniline nachweist.

3. Verfahren nach einem der Ansprüche 1 bis 2, **dadurch gekennzeichnet, dass** das Präsenilin PS2 ist.

4. Verfahren nach einem der Ansprüche 1 bis 3, **dadurch gekennzeichnet, dass** der N-Terminus des Präsenilins der Abschnitt 1-87 von PS2 ist.

5. Verfahren nach einem der Ansprüche 2 bis 4, **dadurch gekennzeichnet, dass** es folgende Schritte umfasst:
- das Peptid Aß₁₋₄₂ wird zuvor durch Inkubation an eine Nitrocellulosemembran absorbiert,
- ein Bakterienextrakt, der ein gesamtes oder einen Teil eines Präsenilins enthält, wird für die Inkubation mit dem zu testenden Molekül oder einem Gemisch, das verschiedene zu testende Moleküle enthält, zugegeben,
- die Wechselwirkung des Präsenilins mit dem Peptid Aß₁₋₄₂ auf dem Nitrocellulosefilter wird mithilfe der Markerproteine der Präseniline nachgewiesen.

6. Verfahren nach Anspruch 5, **dadurch gekennzeichnet, dass** es sich bei einem der Markerproteine um das S-Tag-Bindungsprotein, gekoppelt an alkalische Phosphatase, handelt.
